(19)

Europäisches
Patentamt

European
Patent Office

Office européen
des brevets

(11) **EP 2 891 457 A1**

(12) **EUROPEAN PATENT APPLICATION**
published in accordance with Art. 153(4) EPC

(43) Date of publication:
**08.07.2015 Bulletin 2015/28**

(21) Application number: **13833379.4**

(22) Date of filing: **09.07.2013**

(51) Int Cl.:
**A61B 10/00** (2006.01)     **A61B 5/1455** (2006.01)

(86) International application number:
**PCT/JP2013/068788**

(87) International publication number:
**WO 2014/034285 (06.03.2014 Gazette 2014/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **31.08.2012   JP 2012191039**

(71) Applicant: **Hitachi Medical Corporation
Chiyoda-ku
Tokyo 101-0021 (JP)**

(72) Inventors:
• **FUNANE, Tsukasa
Chiyoda-ku,
Tokyo 100-8280 (JP)**

• **KIGUCHI, Masashi
Chiyoda-ku
Tokyo 100-8280 (JP)**
• **FUJIWARA, Michiyuki
Tokyo 101-0021 (JP)**
• **KAGA, Mikihiro
Tokyo 101-0021 (JP)**
• **TAKATERA, Tsuyoshi
Tokyo 101-0021 (JP)**

(74) Representative: **MERH-IP
Matias Erny Reichl Hoffmann
Paul-Heyse-Strasse 29
80336 München (DE)**

(54) **BIOPHOTONIC MEASUREMENT APPARATUS AND BIOPHOTONIC MEASUREMENT METHOD USING SAME**

(57)     In a biophotonic measurement apparatus using a probe having a plurality of irradiation-detector distances (SD distances) in order to separate light absorption changes in a surface layer and a deep layer of a biological tissue on the basis of near infrared spectroscopy, light reception sensitivity is stabilized on each subject/each region by adjusting an attenuation amount of light to be transmitted or received. It has a light source, a detector for detecting light that has been irradiated from the light source to an irradiation point on the subject and propagated in the subject, a light attenuation amount adjusting means to be disposed on an optical path between the light source - the subject or a photodetector - the subject, an analysis unit for analyzing a signal and a display unit for display a result of analysis, the light source and the detector are respectively arranged such the SD distance defined as a distance between an irradiation point and a detection point is given in two or more kinds, the analysis unit analyzes a measurement signal and calculates a light attenuation adjustment amount for setting respective received light amounts within a predetermined range, and the light attenuation amount adjusting means makes attenuation amounts of light respectively adjustable from

the result of analysis by changing an amount of light that is incident upon the detector.

FIG. 1

**Description**

Technical Field

The present invention relates to a biophotonic measurement apparatus using visible light or near infrared light.

Background Art

There is a report that in a light detection signal and a biological signal (hereinafter, an NIRS signal) obtained by imaging a non-invasive optical brain function using Near-infrared spectroscopy (NIRS) including an optical topography method, since light is irradiated from above the scalp, there is the possibility that it may be influenced by a fluctuation in skin blood flow of the scalp. In consideration of the influence of the skin blood flow like this, methods of extracting/removing components thereof are being studied. Many of them are of the type that signal components from regions that are different in depth are acquired by a system of a plurality of irradiation - detector (light transmitter - light receiver) distances (hereinafter, SD (Source - Detector) distances), and a skin blood flow signal that is thought to influence measurement data on a shallow layer part is removed by using them. In the following, the system of performing measurement over the plurality of SD distances will be called a multi-SD system.

As a skin blood flow removing method utilizing the multi-SD system, conventionally, there is disclosed a method of subtracting the one that a proper scaling factor has been multiplied by a measurement signal over a short SD distance from a measurement signal over a long SD distance, depending on a subject and a measurement region, in order to remove the signal of the skin blood flow and so forth in Patent Literature 1. However, this method presupposes that the multi-SD measurement data can be used, and in a case where a proper signal cannot be acquired over each SD distance, for example, when the detector is saturated because an amount of light is large, it becomes difficult to apply it.

In addition, in Patent Literature 2, there is disclosed a method of irradiating light having a wavelength to be absorbed by a measurement object from a light irradiation unit toward the inside of the subject while changing its intensity with time and detecting the light transmitted through the subject by a plurality of detection elements arrayed along a scanning direction. Although this method is suited for measurement in a one-dimensional direction, it is difficult to apply it to two-dimensional imaging used in brain function measurement and so forth.

Further, in Patent Literature 3, there is disclosed a method of, for the purpose of separating and remove the influence of the skin blood flow included in the NISR signal and extracting a brain or cerebral cortex derived signal, arranging light transmitters and light receivers such that measurement over the plurality of SD distances is implemented and light that each light receiver receives is propagated through the gray matter, performing Independent component analysis (ICA) by using data at each measurement point, and deciding whether each independent component is derived from the brain or the skin by using SD distance dependency of a weighted value of each separated component. Also in this method, that the multi-SD measurement data can be used is set as a presupposition and stable signal acquisition becomes a problem.

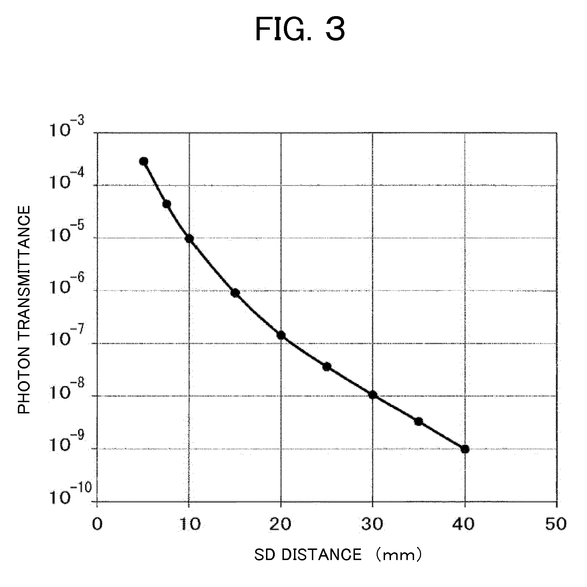

Although there also exist a method of adjusting light reception sensitivity in time division and a method of changing the power of a light source in time division when a certain light transmitter or light receiver is to be commonly used for measurement of the plurality of different SD distances by the multi-SD system as described in Non-Patent Literature 3, since probes are to be highly densely arranged in the multi-SD, there is such a problem that measurement in time division results in deterioration of time resolution. Further, in a case where measurement of, for example, the SD distances 5 mm and 30 mm is simultaneously performed still in a case where measurement is performed in time division, there is a difference in detected light amount of at least four digits as shown in FIG. 3, a highly accurate and multi-stage amplification circuit and so forth become necessary in control by an electromagnetic action, and an increase in production cost is induced.

Further, upon multi-SD measurement, since there exist the plurality of kinds of the SD distances and the measurement points of each SD distance are equally arranged spatially, designing for sensitivity uniformity at each measurement point is made efficient. Also in analysis that the measurement object is to be imaged two-dimensionally or three-dimensionally, uniform distribution of the measurement points is important and appropriate arrangement of the light transmitters and the light receivers for implementing such measurement is needed.

The transmittance of light is different depending on various conditions such as the SD distance, the subject (age and sex), the measurement region and so forth. In particular, in the human forehead, an average optical path length and the transmittance are greatly changed under the influence of a difference in paranasal sinuses (or frontal sinus) among individuals (see Patent Literature 4). In optical brain function measurement by the multi-SD system, it was necessary to adjust the light amount and to cope with it by insertion and so forth of an optical filter or an optical attenuator in accordance with each condition upon measurement. In a case where the optical filter is not used, there was the possibility that appropriate gain setting for the detector cannot be performed against a difference in detected amount of light from a plurality of light sources exceeding the dynamic range of the detector. In a case where the optical filter is used, it is necessary to once remove the optical fiber on the probe side or the apparatus main body side and there was such a problem that it takes time and labor. Further, in a case where one detector simultaneously receives light from the light sources located at positions of the plurality of SD distances, it was necessary to use a detector of a wide dynamic range or to insert the optical filter into the light source side in accordance with the SD distance or the detected light amount in order to stably detect the plurality of signals of different light amounts.

Citation List

Patent Literature

PTL 1: Japanese Patent Application Laid-Open No. 2010-240298
PTL 2: Japanese Patent Application Laid-Open No. 2006-200943
PTL 3: PCT WO/2012/005303
PTL 4: PCT WO/2010/150751

Non-Patent Literature

Non-PTL 1: A. Maki et al., "Spatial and temporal analysis of human mot or activity using noninvasive NIR topography", Medical Physics, Vol.22, No.12, pp. 1997-2005 (1995)
Non-PTL 2: T. Funane et al., "Discrimination of Skin Blood Flow Using Multi-Distance Probe and Independent Component Analysis in Optical Brain Function Monitoring", The papers of Technical Meeting on Optical and Quantum Devices, IEE, Japan, OQD-11-033, pp. 17-22 (2011)
Non-PTL 3: I. Oda et al., "Near Infrared Imager with a Flexible Source-Detector Arrangement and a New Detection Gain Control", Optical Review 10(5), pp. 422-426 (2003)

Summary of Invention

Technical Problem

However, when the optical filter is used in the light transmitter or the light receiver, such a problem arises that a signal to noise ratio (S/N) is small for the long SD distance due to attenuation of light, measurement thereof becomes difficult and that light transmitter or that light receiver can be used only for measurement of the short SD distance. In addition, in a case where measurement of the long SD distance is to be performed by using the probe (the light transmitter or the light receiver) with the optical filter mounted, it is necessary to change the sensitivity of the photodetector with time (see Non-Patent Literature 3) or to change the light amount with time.
The present invention aims to, in a biophotonic measurement apparatus that uses a multi-distance probe having the plurality of irradiation - detector distances in order to separate light absorption changes in a surface layer and a deep layer of a biological tissue on the basis of the Near-infrared spectroscopy (NIRS), stabilize the light reception sensitivity on each subject/ each region by adjusting an attenuation amount of light to be transmitted or received. Solution to Problem
In order to solve the above-mentioned problems, the biophotonic measurement apparatus of the present invention has a light attenuation amount adjusting means and stabilizes the light reception sensitivity by adjusting the attenuation amount of light to be transmitted or received.
Giving one example of the biophotonic measurement apparatus of the present invention, it includes one or a plurality of light irradiation means for irradiating light to a subject, one or a plurality of photo-detection means for detecting light that has been irradiated from the aforementioned light irradiations means to an irradiation point on the aforementioned subject and propagated in the aforementioned subject at a detection point on the aforementioned subject, a light attenuation amount adjusting means disposed on a light propagation path between a light source element included in the aforementioned light irradiation means and the aforementioned subject, or between a light receiving element included in the aforementioned photo-detection means and the aforementioned subject, an analysis unit for analyzing a signal obtained by the aforementioned photo-detection means and a display unit for displaying a result of analysis by the aforementioned analysis, wherein the aforementioned light irradiation means and the aforementioned photo-detection means are respectively arranged on the aforementioned subject such that an SD distance defined as a distance between the aforementioned irradiation point and the aforementioned detection point on the aforementioned subject is given in two or more kinds, the aforementioned analysis unit analyzes signals by light from the aforementioned light irradiation means located at positions of two or more kinds of the SD distances that at least one of the aforementioned photo-detection means detects respectively and calculates a light attenuation adjustment amount for setting respective received light amounts within a predetermined range, and the aforementioned light attenuation amount adjusting means makes attenuation amounts of light from the aforementioned light irradiation means located at the positions of two or more kinds of the SD distances that the aforementioned photo-detection means detects respectively adjustable by changing an amount of light that is incident upon the aforementioned photo-detection means.
A biophotonic measurement method using the biophotonic measurement apparatus of the present invention includes the step of measuring light that has been propagated in a subject at at least one measurement point of less than 10 mm, preferably not more than 8 mm in SD distance, and two or more measurement points of at least 10 mm, preferably at least 12 mm in SD distance,

the step of obtaining a primary straight line using weighted values of two or more measurement points of at least 10 mm in SD distance and obtaining the SD distance corresponding to a weight of zero in a chart that plots the SD distance and a weighted value of each component obtained by a signal separation method, the step of obtaining a straight line that is parallel with an axis using the weighted value of the measurement point of less than 10 mm in SD distance and the step of obtaining the SD distance of an intersection point of the aforementioned straight line that is parallel with the axis and the aforementioned primary straight line as a gray matter reached minimum SD distance and the step of calculating a brain contribution ratio from the SD distance corresponding to the aforementioned weight of zero and the aforementioned gray matter reached minimum SD distance.

Advantageous Effects of Invention

According to the present invention, stabilization of a light reception signal can be implemented by adjusting the attenuation amount of light to be transmitted or received, after consideration of a difference in transmittance among individual subjects and measurement regions. In addition, the attenuation amount of light on a waveguide path of each of the light transmitters and the light receivers can be adjusted automatically or manually by an operator of the apparatus with ease and efficient signal acquisition that has been reduced in temporal and personal costs can be implemented.

Brief Description of Drawings

[Figure 1]

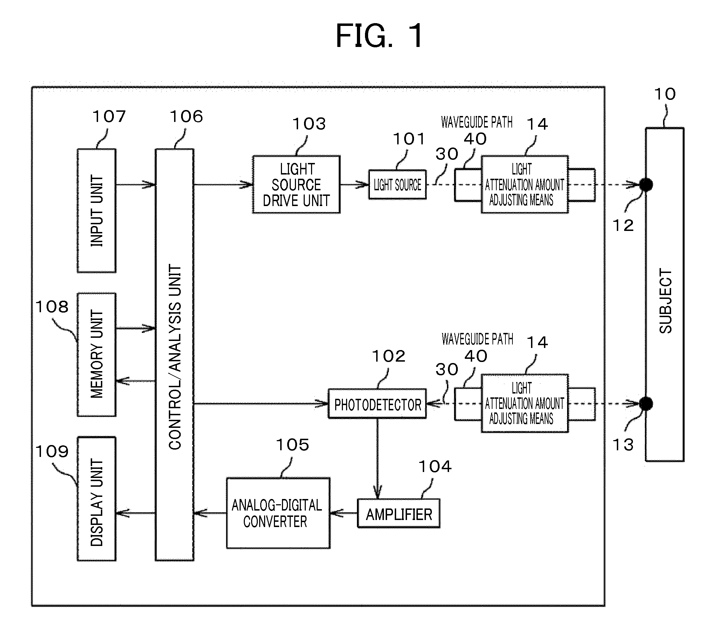

Fig. 1 is a diagram showing one example of an apparatus configuration of a biophotonic measurement apparatus of the present invention.
[Figure 2]

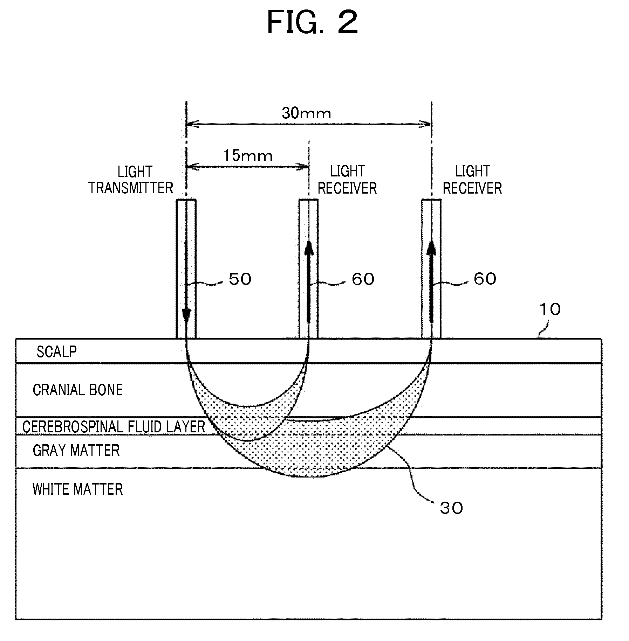

Fig. 2 is a diagram showing an example of a measurement sectional diagram of a multi-SD system.
[Figure 3]
Fig. 3 is a diagram showing a relation between an SD distance and a photon transmittance in a typical head model.
[Figure 4]

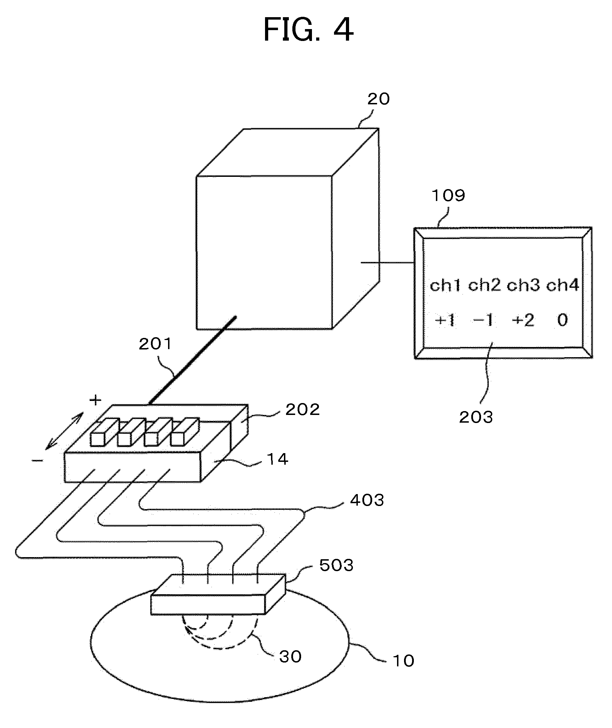

Fig. 4 is a diagram showing an embodiment of the present invention including display of a subject and a light attenuation amount adjustment amount.
[Figure 5]

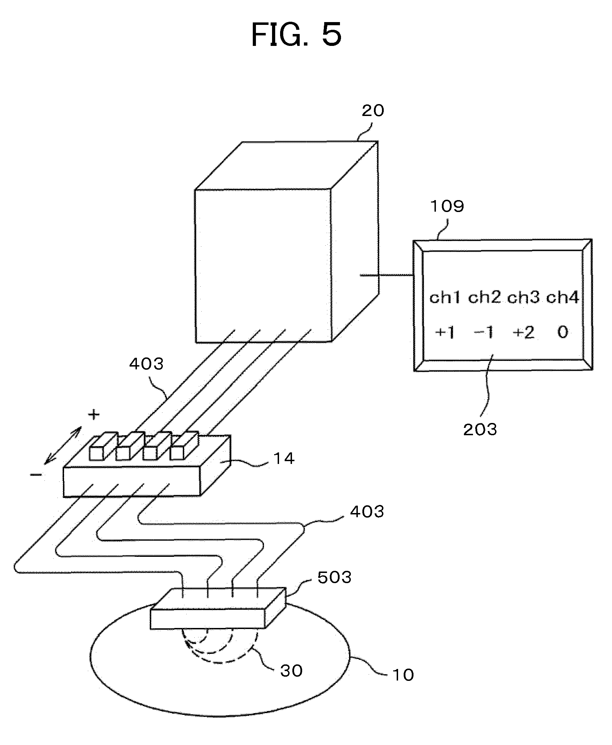

Fig. 5 is a diagram showing another embodiment of the present invention including display of the subject and the light attenuation amount adjustment amount.
[Figure 6]

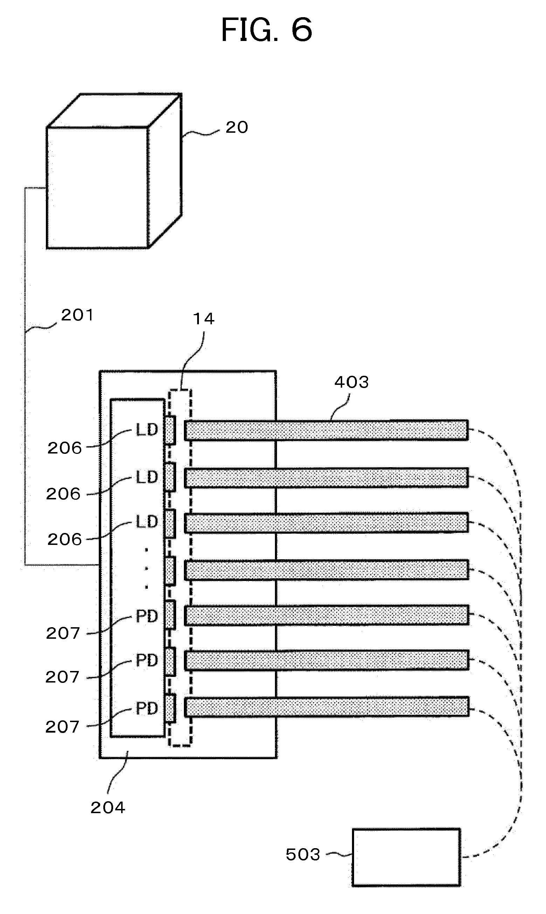

Fig. 6 is a diagram showing a relation between a light attenuation amount adjusting means and surrounding components.

[Figure 7]

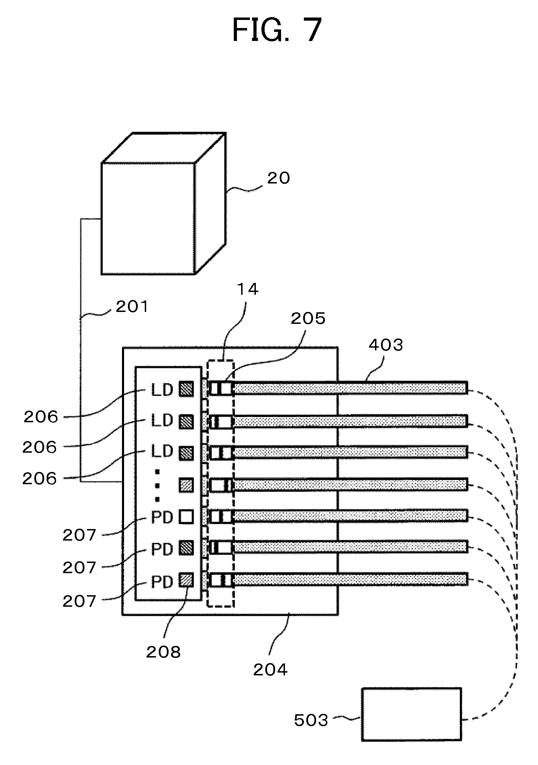

Fig. 7 is a diagram showing a specific example of the light attenuation amount adjusting means and a display unit.
[Figure 8]

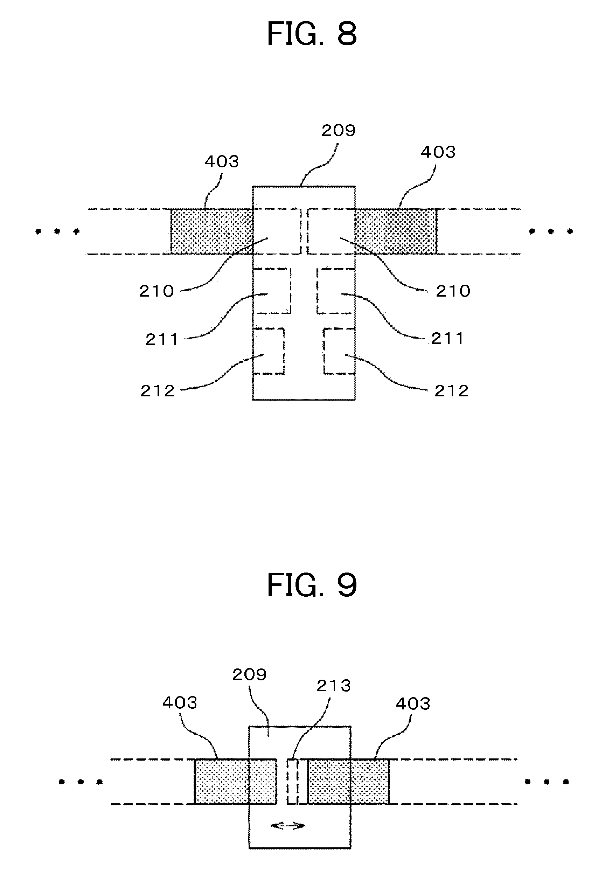

Fig. 8 is a diagram showing the light attenuation amount adjusting means consisting of an intermediate connector having a plurality of insertion ports.
[Figure 9]
Fig. 9 is a diagram showing the light attenuation amount adjusting means consisting of the intermediate connector stepwise insertion into which is possible.
[Figure 10]

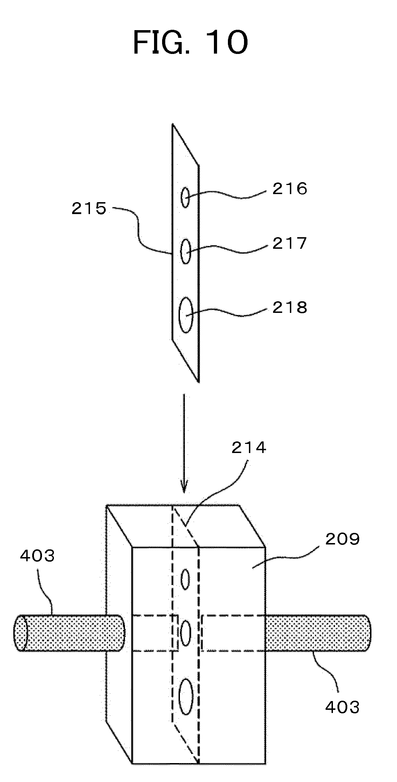

Fig. 10 is a diagram showing the light attenuation amount adjusting means for adjusting the attenuation amount of light depending on the hole size.
[Figure 11]

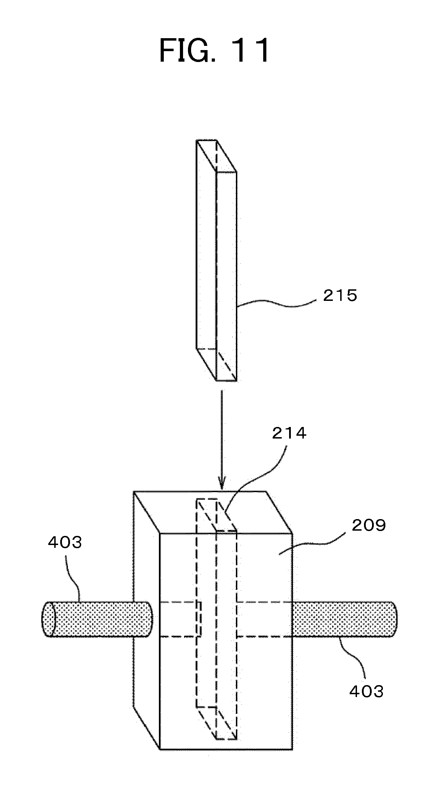

Fig. 11 is a diagram showing the light attenuation amount adjusting means for adjusting the attenuation amount of light by a medium.
[Figure 12]

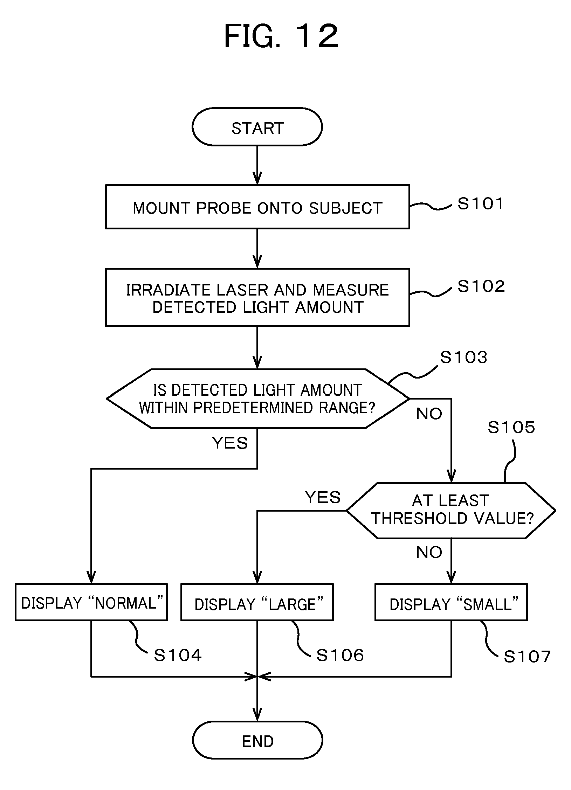

Fig. 12 is a diagram showing a flow for displaying a detected light amount or a control amount.
[Figure 13]

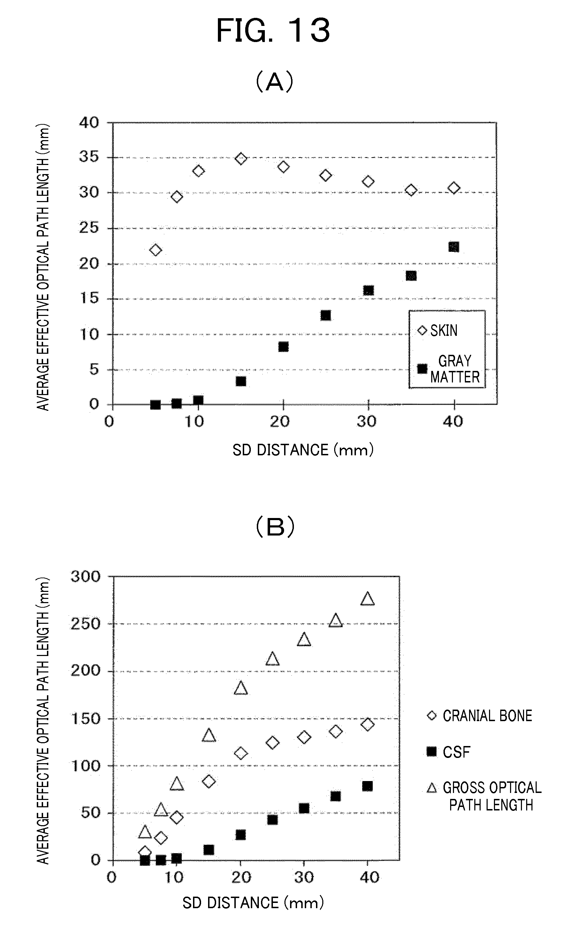

Fig. 13 is diagrams showing a relation between the SD distance and an average optical path length.
[Figure 14]

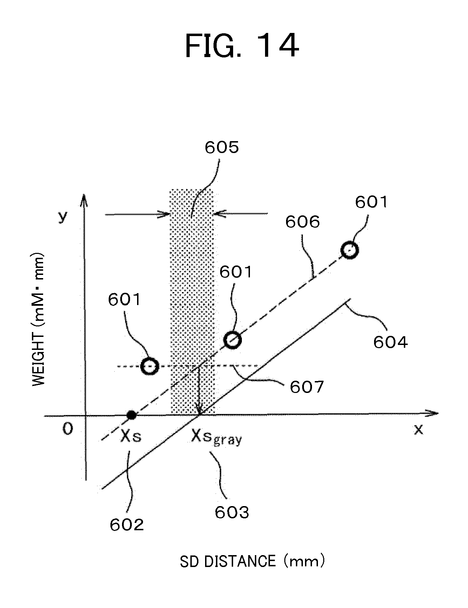

Fig. 14 is a diagram showing a method of estimating a gray matter reached minimum SD distance from measurement data on three kinds of the SD distances.
[Figure 15]

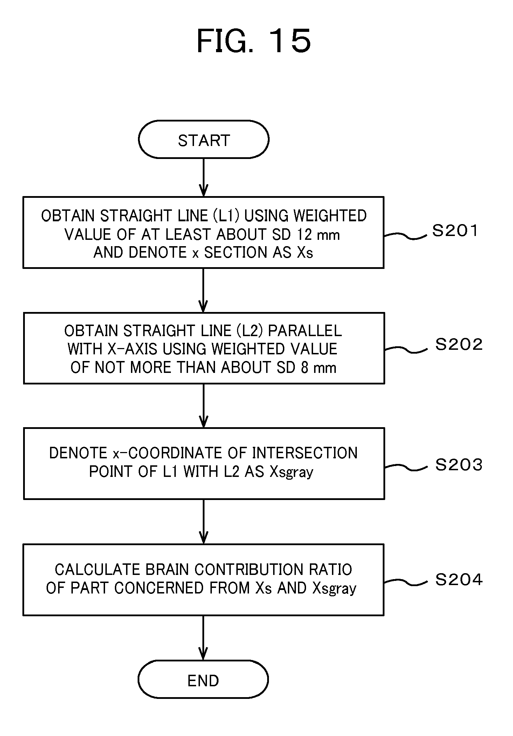

Fig. 15 is a diagram showing a flow for calculating a brain contribution ratio of an NIRS signal by using the gray matter reached minimum SD distance estimated from the measurement data on three kinds of the SD distances.
[Figure 16]

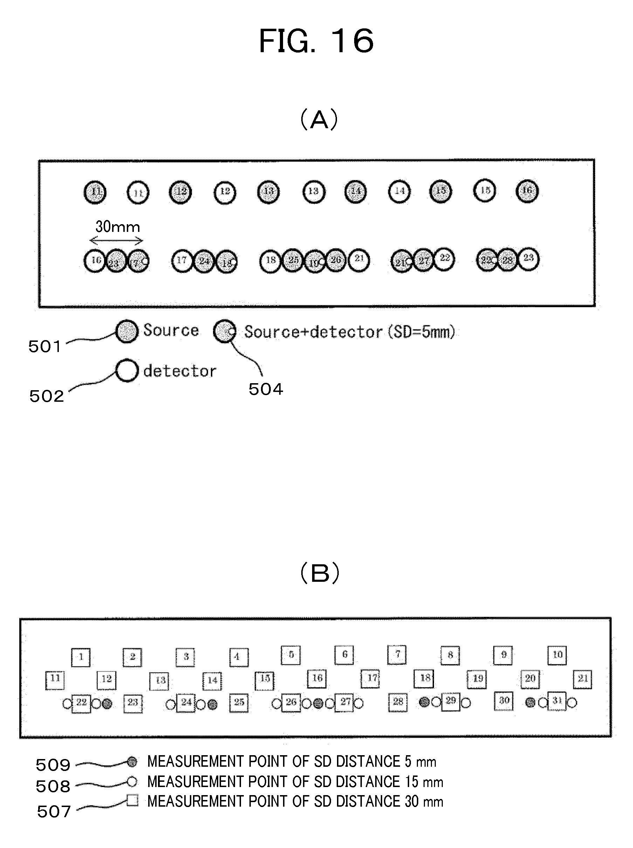

Fig. 16 is diagrams showing (a) a probe arrangement, (b) a measurement point arrangement when the measurement points of the SD distances 5, 15, 30 mm are to be measured on the basis of a 2 X 11 arrangement.
[Figure 17]

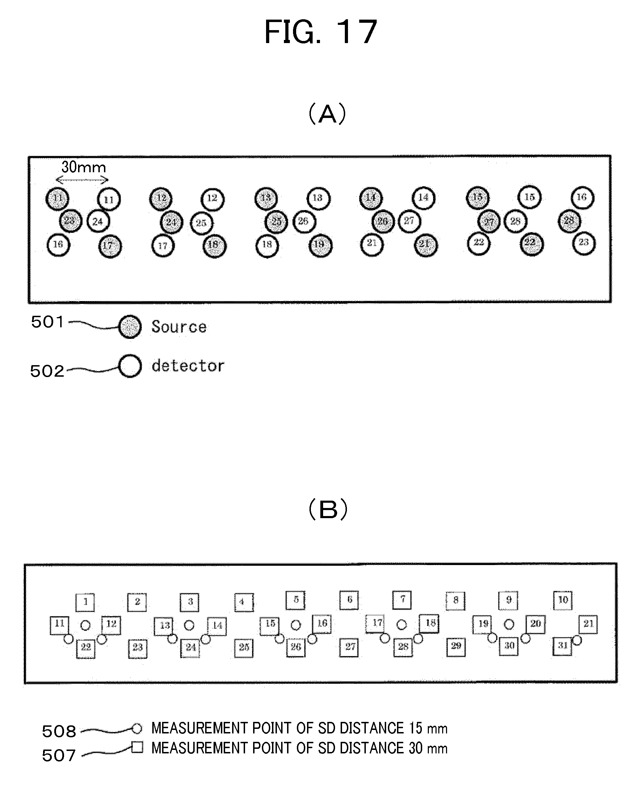

Fig. 17 is diagrams showing (a) a probe arrangement, (b) a measurement point arrangement when the measurement points of the SD distances 15, 30 mm are to be measured on the basis of the 2 X 11 arrangement.
[Figure 18]

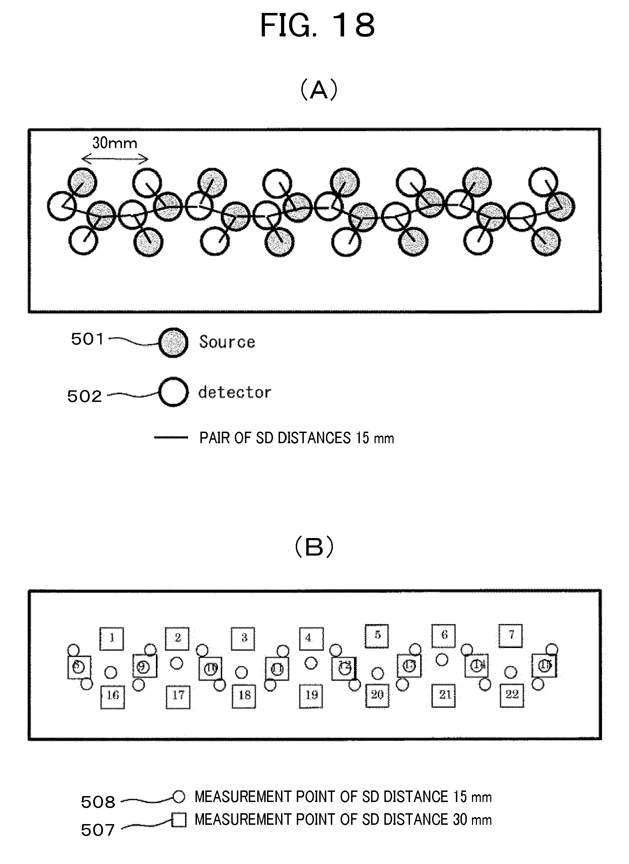

Fig. 18 is diagrams showing (a) a probe arrangement, (b) a measurement point arrangement when the measurement points of the SD distances 15, 30 mm are to be measured respectively at 31 points, 22 points on the basis of a 2 X 8 arrangement.
[Figure 19]

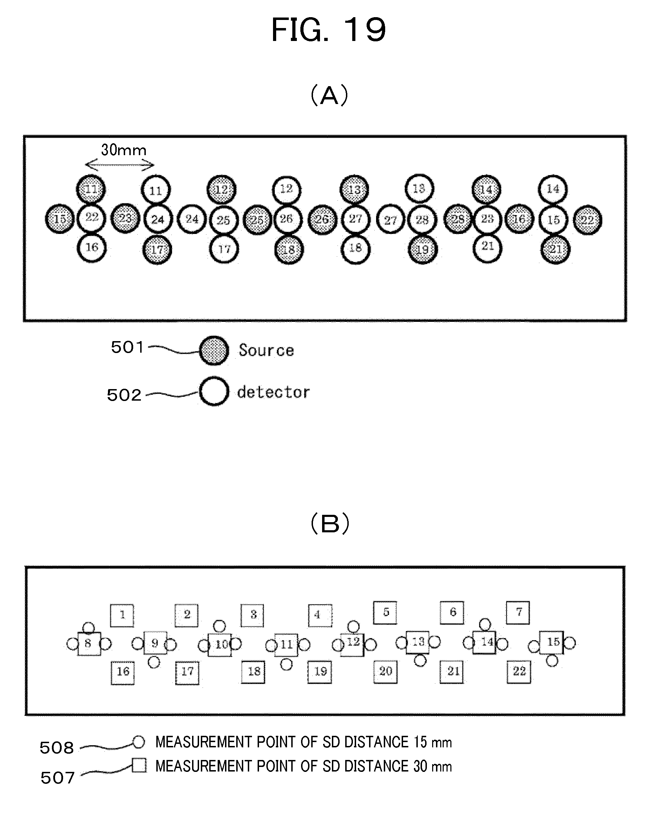

Fig. 19 is a diagram showing (a) a probe arrangement, (b) a measurement point arrangement when the measurement points of the SD distances 15, 30 mm are to be measured respectively at 24 points, 22 points on the basis of the 2 X 8 arrangement.

[Figure 20]

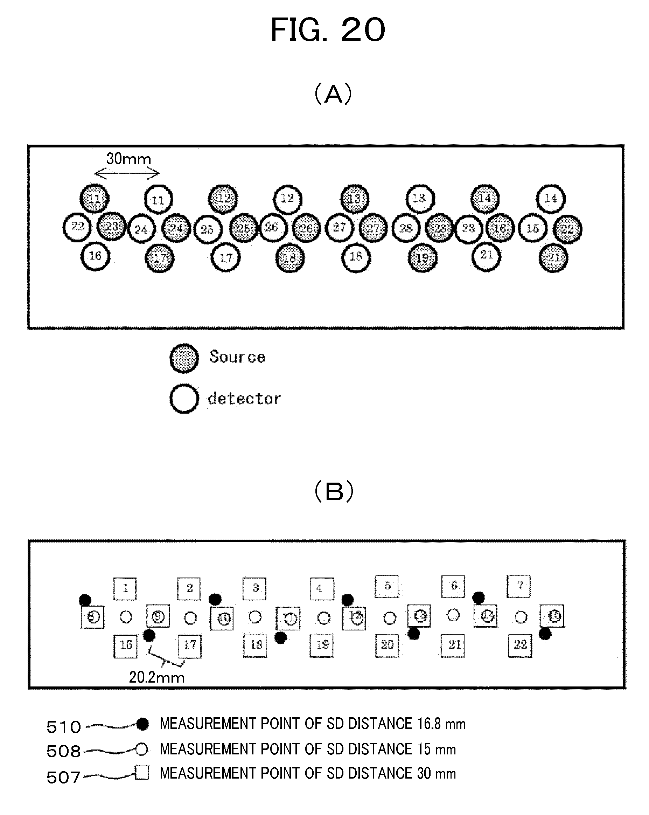

Fig. 20 is diagrams showing (a) a probe arrangement, (b) a measurement point arrangement when the measurement points of the SD distances 15, 16.8 mm, 30 mm are to be measured respectively at 12 points, 8 points, 22 points on the basis of the 2 X 8 arrangement.

[Figure 21]

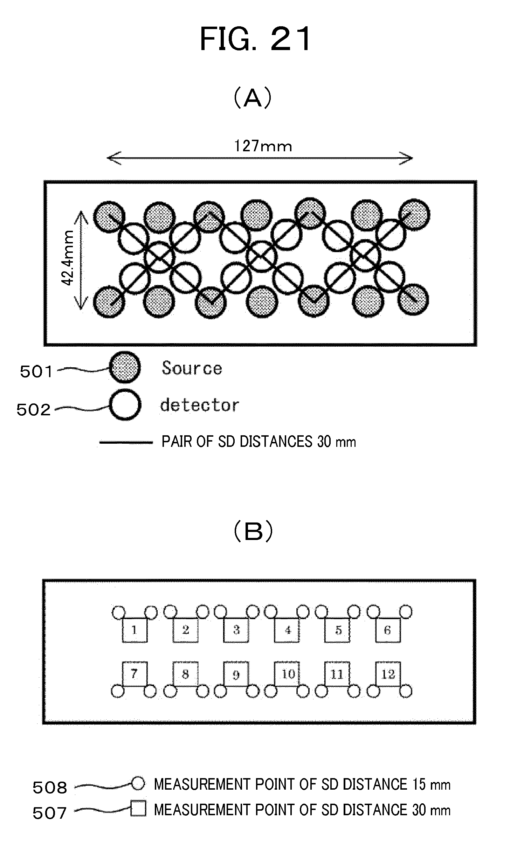

Fig. 21 is diagrams showing (a) a probe arrangement and (b) a measurement point arrangement when the measurement points of the SD distances 15 mm, 30 mm are to be measured respectively at 24, 12 points.

[Figure 22]

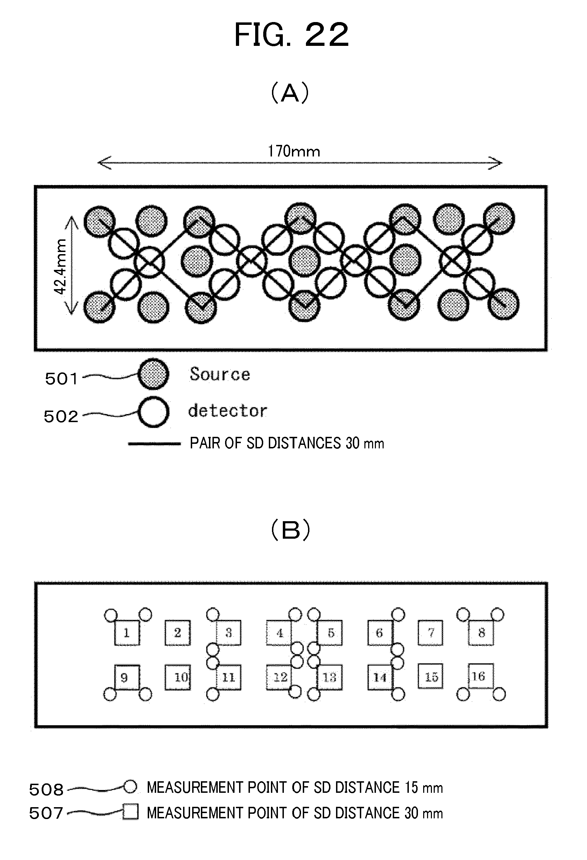

Fig. 22 is diagrams showing (a) a probe arrangement and (b) a measurement point arrangement when the measurement points of the SD distances 15 mm, 30 mm are to be measured respectively at 20, 16 points.

[Figure 23]

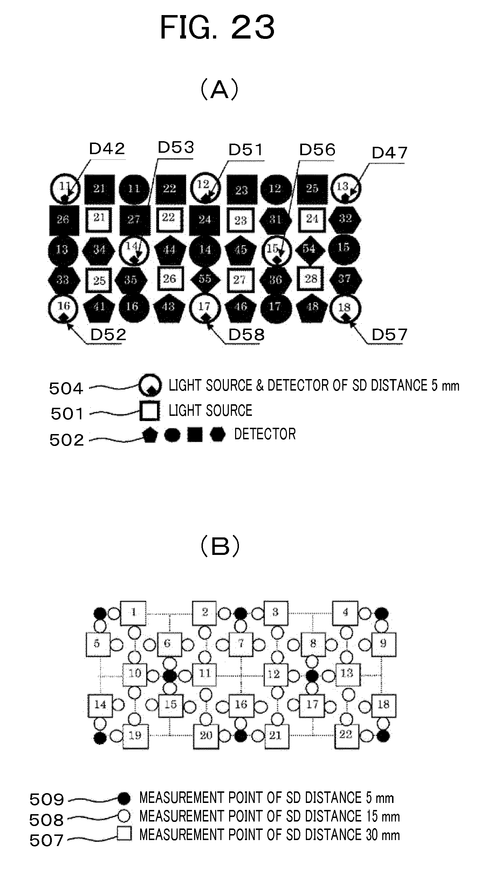

Fig. 23 is diagrams showing (a) a probe arrangement and (b) a measurement point arrangement when the SD distances 5, 15, 30 mm are to be measured on the basis of a 3 X 5 arrangement.

[Figure 24]

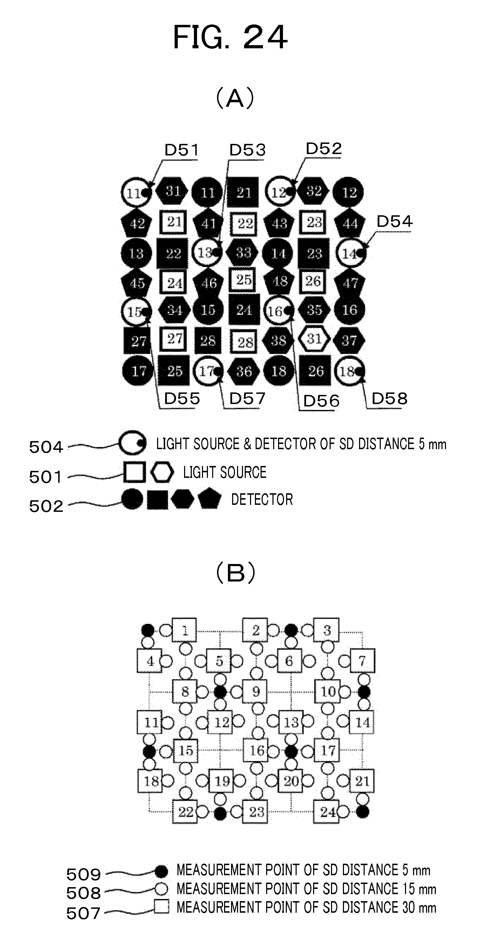

Fig. 24 is diagrams showing (a) a probe arrangement and (b) a measurement point arrangement when the SD distances 5, 15, 30 mm are to be measured on the basis of a 4 X 4 arrangement.

[Figure 25]

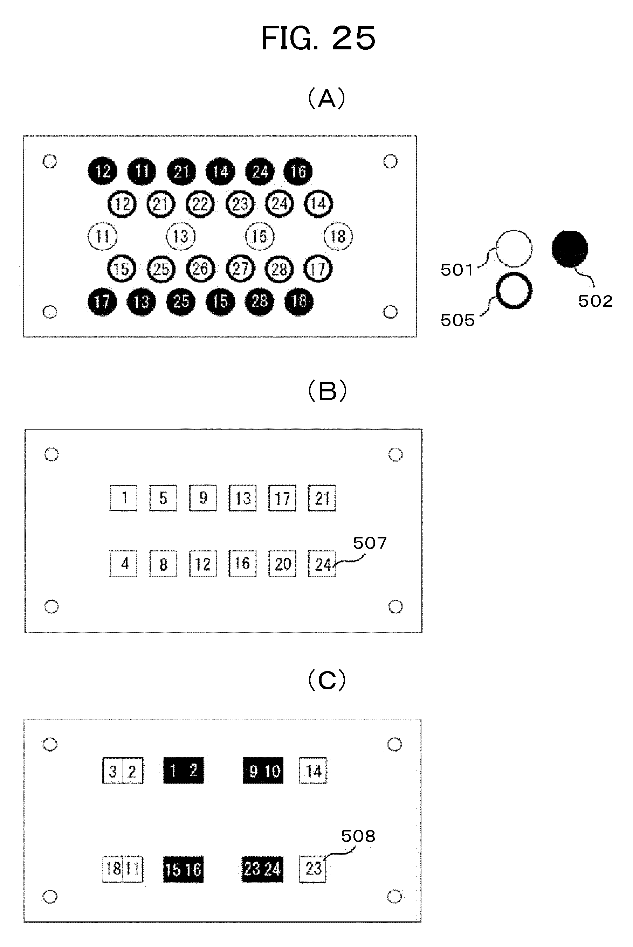

Fig. 25 is diagrams showing (a) a probe arrangement, (b) a measurement point arrangement for the SD distance 30 mm, and (c) a measurement point arrangement for the SD distance 15 mm when the SD distances 15, 30 mm are to be measured (an arrangement A).

[Figure 26]

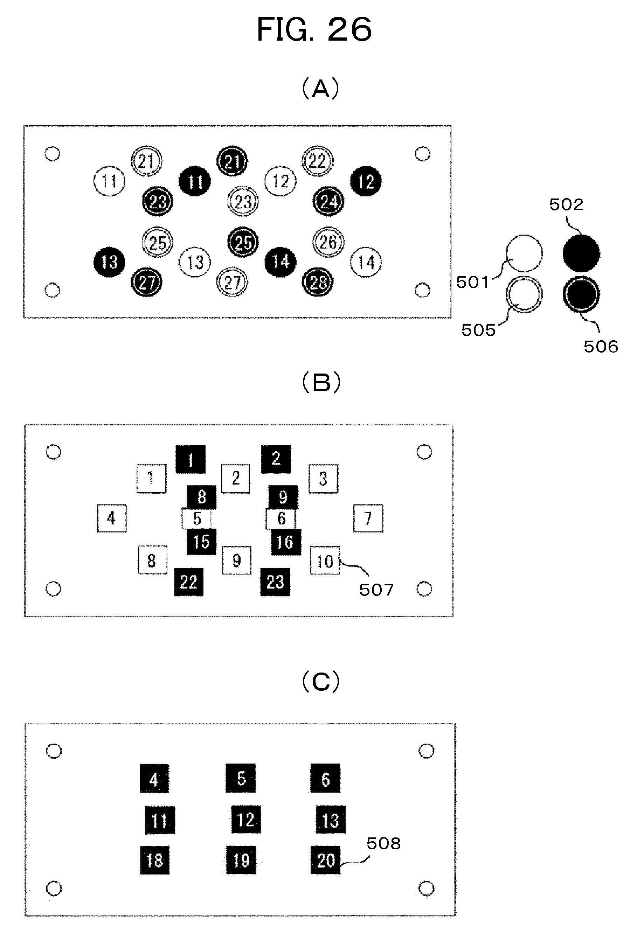

Fig. 26 is diagrams showing (a) a probe arrangement, (b) a measurement point arrangement for the SD distance 30 mm, and (c) a measurement point arrangement for the SD distance 15 mm when the SD distances 15, 30 mm are to be measured (an arrangement B).

[Figure 27]

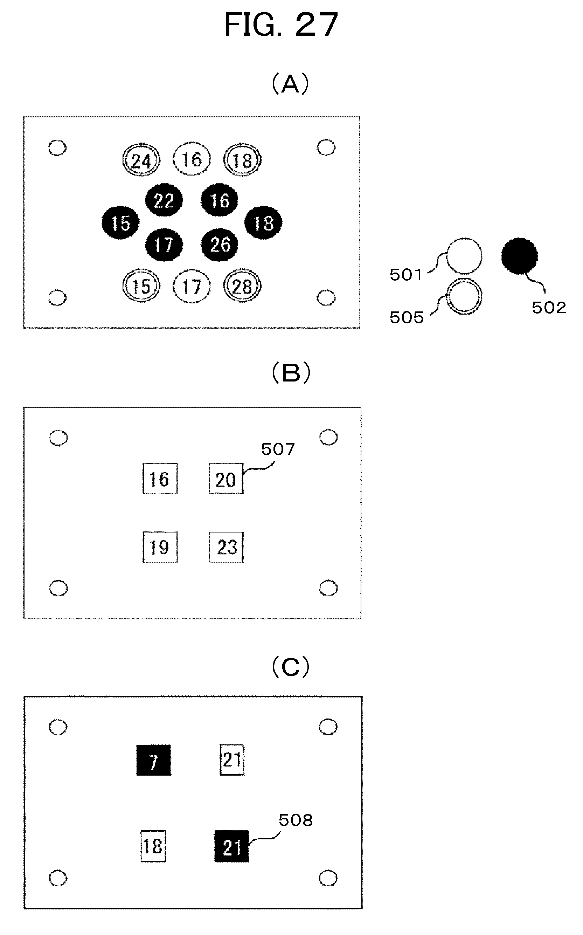

Fig. 27 is diagrams showing (a) a probe arrangement, (b) a measurement point arrangement for the SD distance 30 mm, and (c) a measurement point arrangement for the SD distance 15 mm when the SD distances 15, 30 mm are to be measured (an arrangement C).

[Figure 28]

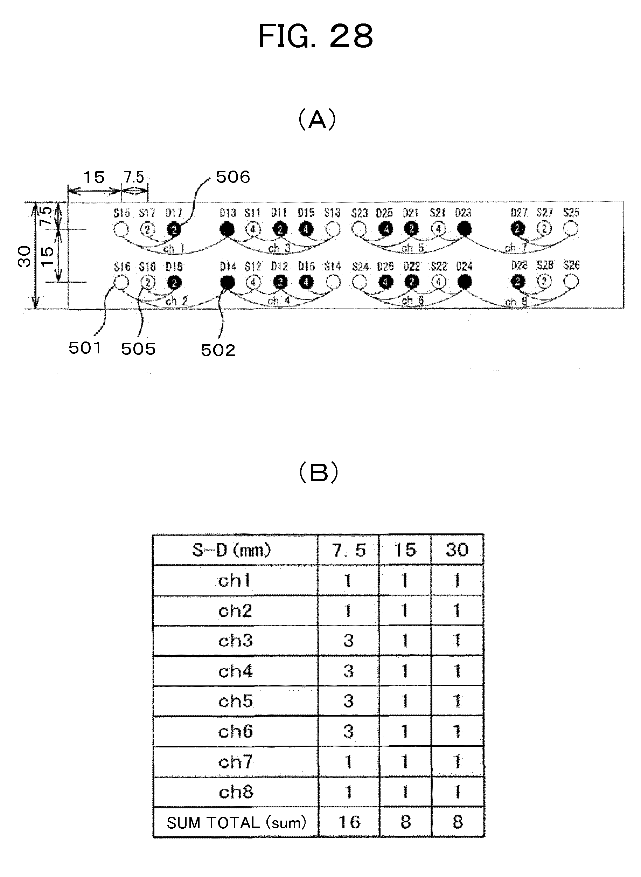

Fig. 28 is diagrams showing (a) a probe arrangement, (b) a table of the number of measurement points of each SD distance in each measurement unit when the SD distances 7.5, 15, 30 mm are to be measured.

[Figure 29]

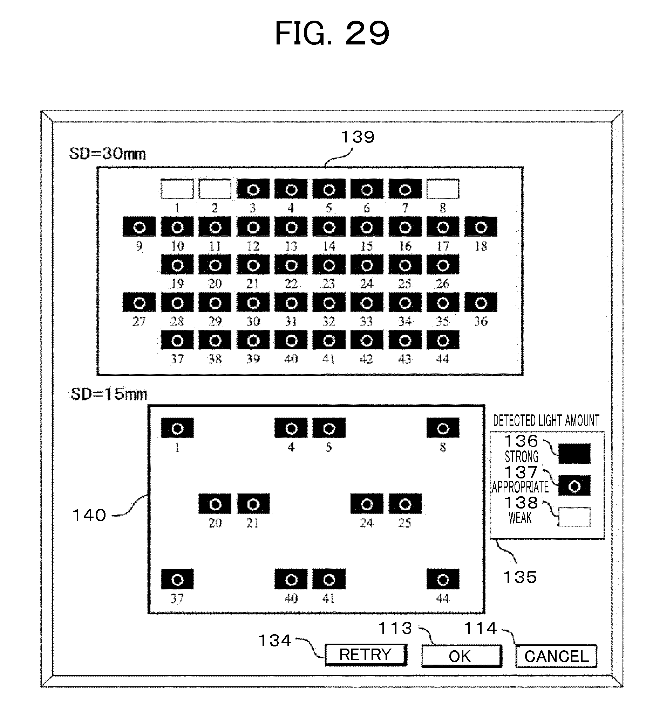

Fig. 29 is a diagram showing a result of a detected light amount at each of the measurement points and the SD distances.

Modes for Carrying Out the Invention

In the following, embodiments of the present invention will be described using the drawings.

First Embodiment

In FIG. 1, one example of an apparatus configuration of a biophotonic measurement apparatus of the present invention is shown. In the biophotonic measurement apparatus that makes light incident upon a living body and detects the light that has been scattered/ absorbed and propagated in the living body and has gone out of it, light 30 irradiated from one or a plurality of light source(s) 101 included in an apparatus main body 20 is made incident upon a subject 10 via a waveguide path 40. The light 30 is incident into the subject 10 from an irradiation point 12, is transmitted and propagated in the subject 10, and is thereafter detected by one or a plurality of photodetector(s) from a detection point 13 located at a position apart from the irradiation point 12 via the waveguide path 40. The SD distance is defined by the distance between the irradiation point 12 and the detection point 13 as aforementioned.

Here, one or the plurality of light source(s) 101 is/are a semiconductor laser(s) (LD), a light emitting diode(s) (LED) and so forth, and one or the plurality of photodetector(s) is/are an avalanche photodiode(s) (APD), a photodiode(s) (PD), a photoelectric multiplier tube (s) (PMT) and so forth. In addition, the waveguide path 40 is an optical fiber, glass, a light guide and so forth.

The light source 101 is driven by a light source drive unit 103 and a gain(s) of one or the plurality of photodetector(s) 102 is/are controlled by a control/analysis unit 106. The control/analysis unit 106 also performs control of the light source drive unit 103 and accepts input of a condition and so forth from an input unit 107.

An electric signal that has been photo-electrically converted by the photodetector 102 is amplified by an amplifier 104, is analog-digital converted by an analog-digital converter 105, is sent to the control/analysis unit 106 and is processed.

In the control/analysis unit 106, analysis is executed on the basis of the signal detected by the photodetector 102. Specifically, it inputs a digital signal obtained by being converted by the analog-digital converter 105 and calculates changes in oxygenated and deoxygenated hemoglobin concentration lengths (oxy - Hb, deoxy - Hd) from a change in detected light amount or a change in absorbance on the basis of, for example, the method described in Non-Patent Literature 1, based on the digital signal concerned. Here, the change in concentration length is an amount of change of the product of the concentration and the optical path length.

Although, here, description has been made on the assumption that the control/analysis unit 106 performs all of driving of the light source 101, gain control of the photodetector 102, processing of signals from the analog-digital converter 105, the same function can be also implemented by respectively having distinct control units and further having a means for integrating them together.

In addition, the measurement data and a result of calculation of the changes in hemoglobin concentration lengths are stored into a memory unit 108 and it is possible to display a result of measurement on a display unit 109 on the basis of a result of analysis and/or the stored data.

Although a light transmitter 50, a light receiver 60 shown in FIG. 2 are not described in FIG. 1, the light transmitter 50 includes, for example, the waveguide path 40 on the light source 101 side and is disposed in contact with the subject 10 or in a nearly contact state, the light receiver 60 includes, for example, the waveguide path 40 on the photodetector 102 side and is disposed in contact with the subject 10 or in the nearly contact state.

In FIG. 2, a situation that light irradiated from the biophotonic measurement apparatus of the present invention is propagated in a biological tissue will be described. It is an example that one light transmitter 50 and two light receivers 60 have been arranged on the scalp. The SD distance is given in two kinds of 15 mm, 30 mm. A penetration depth of irradiated light is made different by a difference in SD distance. That is, the average effective optical path becomes shallow for the SD distance 15 mm and deep for the SD distance 30 mm. It becomes possible to remove the influence of a surface layer signal from a deep layer signal by using the probe so arranged (hereinafter, the light transmitter 50 and the light receiver 60 will be altogether referred to as the probe, and in particular, the probe having the plurality of SD distances is called a multi-distance probe or a multi-SD probe).

In FIG. 3, a relation between the SD distance and a photon transmittance in a typical head model is shown. As shown in FIG. 3, the photon transmittance is exponentially changed with the SD distance. In living body measurement, it is necessary to attain a light amount which can be detected with no saturation of the photodetector 102 and in a sufficient signal to noise ratio. Further, since the tissue structure is different depending on the subject to be measured, the measurement region, the transmittance of light is changed. In order to make the apparatus usable for many subjects and regions, the biophotonic measurement apparatus of the present invention has a light attenuation amount adjusting means 14 shown in FIG. 1 and adjusts the light attenuation amount such that the light amounts detected by the plurality of the aforementioned photodetectors 102 is within a predetermined range.

In FIG. 4, an embodiment of the present invention that includes display of the subject and a light attenuation amount adjustment amount is shown. It is a case where the light source (a semiconductor laser), the photodetector (a photodiode) are included in an outer housing. The Light 30 that the light source 101 in a light source and detector built-in circuit 202 has emitted passes through an optical fiber 403 and is irradiated to the subject 10 from the light transmitter 50 fixed to a probe holder 503. The light 30 that has been propagated in the subject 10 passes through the optical fiber 403 from the light receiver 60 fixed to the probe holder 503 and is received by the photodetector 102 in the light source and detector built-in circuit 202. A light reception signal (a detected light amount) is sent to the apparatus main body 20 as an electric signal 201, whether the detected light amount is within the predetermined range is decided by the not shown control/analysis unit 106, an adjustment amount 203 (relative values such as, for example, +1, -1, +2, 0 and so forth) of the light attenuation amount is displayed on the display unit 109. +1 and so forth express, for example, 10 raised to the power of 1, +2 expresses 10 raised to the power of 2, and the light attenuation amount is made adjustable by automatically or manually operating this much adjustment amount in the light attenuation amount adjusting means 14. In a case where the light attenuation amount is to be automatically adjusted, a control signal is sent from the apparatus main body 20 to the light attenuation amount adjusting means 14 via the electric signal 201. In a case where the light attenuation amount is to be manually adjusted, the operator manually operates the light attenuation amount adjusting means 14 while looking at the display unit 109. Owing to this configuration, the light attenuation amount can be adjusted at a high speed without again attaching/detaching the probe holder 503. Further, there is also such an advantageous effect that burdens on the operator and the subject are reduced. Although display of the relative values has been described here, it may be display of an absolute value. Further, display may be made on the display unit 109 such that a typical light attenuation amount set value or a predetermined set value such as a past set value or the like of the subject concerned can be automatically or manually set in accordance with the region. It becomes possible to easily adjust such large light attenuation that implementation thereof is difficult simply by again attaching/detaching the probe by using the light attenuation amount adjusting means in this way and there is such an advantageous effect that it leads to reductions in measurement time and cost.

In FIG. 5, another embodiment of the present invention including display of the subject and the light attenuation amount adjustment amount is shown. It is a case where the outer housing has an intermediate connector of the optical fibers. Although, in FIG. 4, generation of light, photoelectric conversion of the detected light have been performed in the light source and detector built-in circuit 202, in a configuration of FIG. 5, the light attenuation amount adjusting means 14 is arranged on a joint part(s) of one or both of the optical fibers 403 so as to be interposed between the optical fibers 403 on the apparatus main body side and on the probe holder side. The light attenuation amount adjusting means 14 may be arranged on either a light transmission fiber or a light reception fiber. The light attenuation amount adjusting means 14 adjusts the amount of light that is propagated through the optical fiber 403 by optical actions such as scattering, absorption, reflection and so forth of light. Other configurations are the same as those of the case in FIG. 4. Owing to this configuration, the light attenuation amount adjusting means 14 can be added to the optical fiber of the existing apparatus as an independent module.

Next, the light attenuation amount adjusting means 14 corresponding to that in FIG. 4 will be described by using FIG. 6. In FIG. 6, a relation between the light attenuation amount adjusting unit and surrounding components is shown. In the housing 204, the light attenuation amount adjusting means 14 is disposed between an optical element such as a semiconductor laser 206 or a photodiode 207 and so forth arranged in the same housing 204 and the optical fibers 403 and individually adjusts connection losses between them and the optical fibers 403 connected to the respective elements. Here, as an electromagnetic means, an automatically or manually adjustable piezoelectric actuator or piezoelectric element is provided so as to minimally change an end face positional relation between the optical fibers 403. There is such an advantageous effect that light reception sensitivity adjustment can be performed at a position closer to the subject 10 simultaneously with probe mounting and measurement can be made efficient, by putting the optical elements and the light attenuation amount adjusting means 14 together into the outer housing 204 that is different from the apparatus main body 20 in this way. Incidentally, although the electromagnetic means has been described here, it may be a mechanical means using a motor, a gear wheel, or a rack-pinion mechanism, hydraulic pressure, pneumatic pressure and so forth. In addition, it may be either automatic or manual means.

In FIG. 7, a specific example of the light attenuation amount adjusting means (a manual adjustment knob) and the display unit (color display by light emitting diodes) is shown. It is of the type that the configuration in FIG. 6 has been more embodied, the light attenuation amount adjusting means 14 has a manual adjustment knob 205 and further the adjustment amount has been indicated by a color-dependent display 208 as the display unit 109. Incidentally, on the color-dependent display 208, shades of white and black or a predetermined color, the length of an arrow indicating a direction of the knob and so forth may be displayed. The biophotonic measurement apparatus of this configuration has such an advantageous effect that an operating method is simplified and errors when the operator operates it can be prevented by having the display unit 109 in the same housing 204 that the manual adjustment knob 205 that the operator operates is included. Further, since a result of operation can be informed of to the operator soon, there is an advantageous effect that measurement is made efficient.

In FIG. 8, a structure of an intermediate connector for optical fiber connection that has a plurality of insertion ports is shown as one example of the light attenuation amount adjusting means. An intermediate connector 209 is used to optically coupling together the two optical fibers 403 and is provided with two ports each as an optical fiber insertion port (the attenuation amount is small) 210, an optical fiber insertion port (the attenuation amount is medium) 211, an optical fiber insertion port (the attenuation amount is large) 212 so as to mutually face, and the distance between the optical fiber end faces and the positional relation between them are changed by inserting the optical fibers 403 into the both sides one by one, and thereby it becomes possible to adjust the attenuation amount of light in the optical fiber 403 concerned. Owing to this configuration, it becomes possible to easily adjust the attenuation amount of light manually. It becomes also possible to adjust the attenuation amount of light in a wider range by increasing the number of kinds of the respective insertion ports in the intermediate connector.

In FIG. 9, a structure of the intermediate connector into which it can be inserted stepwise is shown as another example of the light attenuation amount adjusting means. The intermediate connector 209 has an insertion depth adjusting part 213 and can adjust the interface-end distance between the optical fibers 403 in several steps. Here, a method of continuously adjusting the inter-end-face distance by providing a screw-shaped structure and while rotating it may be also adoptable, in place of stepwise insertion of the optical fiber 403. Owing to this configuration, adjustment of the light attenuation amount becomes possible in a smaller apparatus.

In FIG. 10, a mechanism for adjusting the light attenuation amount depending on the hole size is shown as still another example of the light attenuation amount adjusting means. The intermediate connector 209 in FIG. 10 has an insertion port 214. A medium or a metal member having a waveguide hole (the attenuation amount is large) 216, a waveguide hole (the attenuation amount is medium) 217, a waveguide hole (the attenuation amount is small) 218 is inserted through the insertion port 214 and the waveguide holes that are different from one another in hole diameter are arranged on the waveguide path of light that is propagated through the optical fiber 403, thereby controlling the light attenuation amount. This operation may be either automatic or manual.

In FIG. 11, a mechanism for adjusting the light attenuation amount by a medium is shown as still another example of the light attenuation amount adjusting means. An absorbing or scattering medium 215 is inserted into the intermediate connector 209 in FIG. 11 in place of the medium having the holes in FIG. 10. A plurality of the absorbing or scattering media are prepared and the one corresponding to the light attenuation amount to be implemented is used. This operation may be either automatic or manual. The medium used here may be the one that an absorbing substance such as a dye and so forth and a scattering substance such as titanium oxide and so forth have been mixed into an epoxy resin and also may be a medium that exhibits gradation of concentration of each substance such that optical properties are smoothly changed depending on the position on a predetermined plane in the same member. The light attenuation amount can be adjusted by adjusting the concentrations of the absorbing substance and the scattering substance. Further, a degree of light transmission may be adjusted by disposing a liquid crystal screen that is electromagnetically adjustable and can change the transmittance of light, or dimming glass and a dimming film made of an electrochromic material on the optical path and forming a pattern of appropriate size and shape by switching ON/OFF of each pixel, in place of the medium used here. Further, a thermochromic material which can control the transmittance depending on heat or temperature may be used together with a temperature control means.

Owing to these configurations in FIGS. 10, 11, there is such an advantageous effect that an optional light attenuation amount can be implemented within a wide range and the stable light reception sensitivity can be obtained not depending on the subject, the measurement region.

In FIG. 12, a flowchart for displaying the detected light amount or the adjustment amount is shown. Procedures for displaying the adjustment amount according to a First Embodiment will be described with reference to the flowchart in FIG. 12. First, the probe is mounted onto the subject 10 (step S101). Next, the laser is irradiated to measure the detected light amount (step S102). Whether the detected light amount is within a range of predetermined light amounts (for example, 0.1 to 2 V after A/D conversion) is decided (step S103). In a case where it has been decided that the detected light amount is within the range of 0.1 to 2 V (YES), since the detected light amount is appropriate, "NORMAL" is displayed on the display unit (step S104) . In a case where the detected light amount is less than 0.1 V or larger than 2 V in step S103 (NO), the detected light amount is not appropriate and therefore whether it is larger than a threshold value (for example, 2 V) is further decided (step S105). In a case where it has been decided that the detected light amount is larger than 2 V (YES), it is necessary to reduce the detected light amount and therefore "LARGE" is displayed in order to indicate it (step S106). In a case where it has been decided that the detected light amount is not more than 2 V (NO), the detected light amount is less than 0.1 V in conjunction with the decision in step S103 and therefore it is necessary to increase the detected light amount and "SMALL" is displayed in order to indicate it (step S107). In these displays, results of decision in steps S103 and S105 may be displayed by a difference in color. Further, to what extent it is large or small may be stepwise displayed using numerical values (for example, +1, +3, -2 and so forth) or by the difference in color. Further, these may be displayed as the adjustment amounts by the light attenuation amount adjusting means.

According to the present embodiment, in the biophotonic measurement apparatus using the multi-distance probe having the plurality of irradiation - detector distances in order to separate changes in light absorption in the surface layer and the deep layer of the biological tissue on the basis of the Near-infrared spectroscopy (NIRS), the light reception sensitivity can be stabilized on each subject/each region by adjusting the attenuation amount of light to be transmitted or received. In particular, when the measurement points of the plurality of SD distances are mixed as in cases of measurement using two photodetectors for one light source, measurement using two light sources for one photodetector, and measurement that the both are mixed, the light reception sensitivity can be stabilized by preventing interference between the respective measurement points. When measuring by using the common light source or photodetector in this way, by using the plurality of measurement points which are different from one another in SD distance, it is difficult to cope with a large signal amplitude ratio which would generate in the multi-SD measurement by power adjustment of the commonly used light source and gain adjustment of the photodetector, while in the present embodiment, it becomes possible to adjust the attenuation amounts of light on the waveguide paths of each light source and each photodetector automatically or manually by the operator and efficient signal acquisition can be implemented.

Second Embodiment

Next, a calculation method for the brain contribution ratio using the multi-SD measurement data to be performed by the control/analysis unit 106 will be described. In Patent Literature 3, description is made on the separation method for the brain and skin derived signals, utilizing the signal amplitude or the SD distance dependency of the weight of the component obtained by the signal separation method. In FIG. 13, relations (a) between the SD distance and partial average optical path lengths of the scalp and the gray matter, (b) between the SD distance and the partial average optical path lengths of the cranial bone, the cerebrospinal fluid layer (CSF) and a gross optical path length that have been obtained by Monte Carlo simulation are respectively shown. The horizontal axis is the SD distance (mm) and the vertical axis is the optical path length (mm). In NIRS measurement, since the SD distance influences the penetration depth of the irradiated light, the depth of a target biological tissue can be changed by changing the SD distance. In multi-SD measurement, since shallow part and deep part signals are obtained with different weights over each SD distance, it becomes possible to calculate contribution of the signal derived from each layer by the signal separation method by acquiring data at the plurality of points. Specifically, although no strong SD distance dependency is observed on the partial optical path length of the scalp, linear SD distance dependency is observed on the gray matter. This method is based on such a principle that the NIRS signal intensity is proportional to the partial optical path length of a region where a blood flow change occurs (for example, see Non-Patent Literature 1) and it is expected that when SD distance is increased, the a brain-derived component in the NIRS measurement signal becomes large, while a skin-derived component does not change.

In FIG. 14, a method of estimating a gray matter reached minimum SD distance from measurement data on three kinds of the SD distances is geometrically shown. Multi-SD measurement data is needed for application of this separation method and it becomes possible to obtain a stable signal by the light attenuation amount adjusting means described in the First Embodiment also by such measurement. In analysis using the measurement data, independent components analysis is applied to the NIRS data measured at one measurement point of the SD distance that is not more than 8 mm and two measurement points of the SD distance that is at least 12 mm and a weight 601 of the obtained independent component is plotted relative to the SD distance. At this time, representative amplitude such as the amplitude and so forth of the NIRS signal that a predetermined cognitive task is being performed may be plotted without using the signal separation method. A model of the average effective optical path length shown in FIG. 13(a) is assumed and it is assumed that the average effective optical path length of the skin has a fixed value approximately after the SD distance that is about 8 mm and the average effective optical path length of the gray matter changes primary-functionally after the SD distance that is about 10 mm. Further, when it is assumed that each separated component is influenced (a mixed component) by both of changes in blood flow in the gray matter (the brain) and the skin (the scalp), the amplitude of the signal to be obtained is expressed by superposition that the optical path lengths of the both layers have been weighted with a change in concentration of each layer and therefore it is expected that it has the fixed value from about 8 mm to about 10 mm in SD distance and is linearly increased after about 10 mm (a gray matter reached minimum SD distance 603). A method of estimating the gray matter reached minimum SD distance 603 from the measurement data by utilizing qualitative characteristics to the SD distance dependency of the mixed component like this will be described in the following.

First, in FIG. 14, as for the measurement point of the SD distance that is not more than 8 mm, a straight line (a straight line that is parallel with an x-axis, L2) 607 having an inclination of zero degrees is drawn, a primary straight line (L1) 606 is applied to the measurement point of the SD distance that is at least 12 mm and the x-coordinate of an intersection point of these two straight lines is expressed as $Xs_{gray}$ (the gray matter reached minimum SD distance 603). A straight line that passes this gray matter reached minimum SD distance 603 on the x-axis and is the same as the aforementioned primary straight line 606 in inclination is a straight line 604 that is proportional to the average effective optical path length of the gray matter and the brain contribution ratio over each SD distance can be calculated from an amplitude ratio of the aforementioned primary straight line 606 to the straight line 604. At this time, although the SD distances to be used are not more than 8 mm at one point and at least 12 mm at two points, almost the same analysis is possible even when they are selected from those that are less than 10 mm at one point and at least 10 mm at two points. However, since the gray matter reached minimum SD distance 603 is around 10 mm in a case of a standard person, more generally speaking, it is necessary to select one point from the SD distances that light does not reach the gray matter and two points from the SD distances that light reaches the gray matter. A non-use SD distance zone 605 that is set here is close to 0 mm in average effective optical path length of the gray matter and is small in change in average effective optical path length of the skin in FIG. 13(a). Although, for example, 8 to 12 mm are set, since it becomes an important parameter relevant to the accuracy in calculation of the brain contribution ratio, it becomes important to utilize MRI structure data or X-ray CT (Computed tomography) data and so forth of each subject in order to determine a more accurate width.

Owing to the configuration described here, although it has been necessary to assume the gray matter reached minimum SD distance 603 in the conventional method, it becomes possible to estimate it per subject, per region by actual measurement and higher accurate brain contribution ratio calculation can be expected. Incidentally, although a method using independent component analysis has been described as the signal separation method here, it may be methods using principal component analysis, factor analysis and so forth. In addition, it may be a method using the detected light amount or the representative amplitude of a change in hemoglobin, without using the signal separation method.

In FIG. 15, a flowchart for calculating the brain distribution ratio of the NIRS signal using the gray matter reached minimum SD distance $Xs_{gray}$ estimated from the measurement data on three kinds of the SD distances is shown. The straight line (L1) is calculated using the weighted value of SD that is about 12 mm or more and an x section is expressed as $Xs$ (step S201). Next, the straight line (L2) that is parallel with the x-axis is calculated using the weighted value of SD that is about 8 mm or less (step S202). Next, the x coordinate of the intersection point of L1, L2 is expressed as $Xs_{gray}$ (step S203). Finally, the brain distribution ratio of the component concerned is calculated from $Xs$ and $Xs_{gray}$ (step S204). When the brain distribution ratio (r) is to be calculated, for example, as described in Non-Patent Literature 2, the formula

$$r = (x - X s_{gray})/(x - X s)$$

is used. This is the one that the amplitude ratio of the aforementioned primary straight line 606 to the straight line 604 has been expressed by the formula.

An example of a specific probe arrangement for implementing the multi-SD measurement to which the light attenuation amount adjusting means 14 of the present invention can be effectively applied will be described in the following by using the drawings. Incidentally, the probe arrangement is not limited to the one shown here and may be the one that measurement is possible over the plurality of SD distances, and all probe arrangements are conceivable in accordance with the object of each user.

In FIG. 16, (a) a probe arrangement, (b) a measurement point arrangement when the SD distances 5, 15, 30 mm are to be measured on the basis of a 2 X 11 arrangement are shown.

In FIG. 17, (a) a probe arrangement, (b) a measurement point arrangement when the SD distances 15, 30 mm are to be measured on the basis of the 2 X 11 arrangement are shown.

In FIG. 18, FIG. 19, FIG. 20, (a) a probe arrangement, (b) a measurement point arrangement when the SD distances 15, 30 mm are to be measured on the basis of a 2 X 8 arrangement are shown. These are arrangements that have considered such that the measurement points of each SD distance are distributed as uniformly as possible spatially.

In FIG. 21, (a) a probe arrangement, (b) a measurement arrangement when a pair of the light source and the detector of the SD distance 30 mm is arranged on the vertex of a rhombus (or on the vertex of a square inclined by 45 degrees relative to a horizontal line), the probes are arranged side by side such that the rhombuses of the same shape are in contact with one another in a row and the measurement points of the SD distances 15 mm, 30 mm are to be measured respectively at 24, 12 points are shown. Although a measuring area of the arrangement in FIG. 21 is narrowed in comparison with those of the arrangements in FIG. 16 to FIG. 20, two measurement points of the SD distance 15 mm are present on each measurement point of the SD distance 30 mm and further distances between those measurement points are all as close as 7.5 mm. Thus, it is suited for measurement in a narrow range.

In FIG. 22, (a) a probe arrangement, (b) a measurement point arrangement when the pair of the light source and the detector of the SD distance 30 mm is arranged on the vertex of the rhombus (or on the vertex of the square inclined by 45 degrees relative to the horizontal line), the probes are arranged side by side such that the rhombuses of the same shape are in contact with one another in a row and the measurement points of the SD distances 15 mm, 30 mm are to be measured respectively at 20, 16 points are shown.

In FIG. 23, (a) a probe arrangement, (b) a measurement point arrangement when the SD distances 5, 15, 30 mm are to be measured on the basis of a 3 X 5 arrangement are shown.

In FIG. 24, (a) a probe arrangement, (b) a measurement point arrangement when the SD distances 5, 15, 30 mm are to be measured on the basis of a 4 X 4 arrangement are shown.

In FIG. 16 to FIG. 24, in an area to be measured by the respective light transmitters - light receivers, a probe for light transmission 501, a probe for light reception 502, an integrated type probe 504 of the probe for light transmission and the probe for light reception are arranged such that measurement points 507 of the SD distance 30 mm, measurement points 508 of the SD distance 15 mm, measurement points of the SD distance 5 mm are arranged as uniformly as possible. In respective measurement regions, since the area having a predetermined range is defined as a measurement unit and is analyzed by using simultaneously the data on each SD distance in the measurement unit, it is favorable to arrange the measurement points of each SD distance at positions that are as close as possible to one another in the measurement unit. In this case, a certain SD distance (for example, 30 mm) is defined as a priority SD distance, and arrangement of the probes for light transmission 501, the probes for light reception 502 is determined such that the measurement points (called subsidiary measurement points) of the other SD distances are arranged in a predetermined range (for example, within 20 mm), a distance from an area measured by the light transmitter 50, the light receiver 60 corresponding to the SD distance concerned or a representative position (for example, a middle point position on a surface of the subject 10 between the light transmitter 50 and the light receiver 60, this is referred to as a priority measurement point). Since it is desirable that the predetermined range here be analyzed at the measurement points that are measuring the same range, it is desirable that it be as small as possible (that is, a distance between the priority measurement point and the subsidiary measurement point is small). However, a case where it is unavoidable to arrange them apart from each other due to a limitation and so forth on the number of probes of the apparatus is also conceivable, and in this case it is set within 40 mm and so forth. In FIG. 16 to FIG. 24, maximum values of inter-measurement-point distances of the different SD distances are respectively 33.5 mm, 30.1 mm, 17 mm, 16.8 mm, 15 mm, 7.5 mm, 19 mm, 15.81 mm, 15. 81 mm. In addition, the numbers of the measurement points of the SD distances 5 mm, 15 mm, 30 mm are respectively 5, 12, 31 points in FIG. 16, are respectively 8, 54, 22 points in FIG. 23, are 8, 60, 24 points in FIG. 24. The numbers of the measurement points of the SD distances 15 mm, 30 mm are respectively 16, 31 points in FIG. 17, are respectively 31, 22 points in FIG. 18, are respectively 24, 22 points in FIG. 19, are respectively 24, 12 points in FIG. 21, are respectively 20, 16 points in FIG. 22, are respectively 8, 54, 22 points in FIG. 23, are respectively 8, 60, 24 points in FIG. 24. The numbers of the measurement points of 15 mm, 30 mm and the number of the measurement points 510 of the SD distance 16.8 mm are respectively 12, 22, 8 points in FIG. 20.

Incidentally, even in a case of the probe arrangement of only the SD distances 15 mm, 30 mm, it may be configured that, for example, one detector is added so as to add one measurement point of 5 mm. This measurement point of the SD distance 5 mm can be used for evaluation of the separation performance after separation of the brain-, skin-derived signals. In addition, when it has been assumed that the skin blood flow is uniform in a target measurement range, the measurement point of the SD distance 5 mm can be utilized as a reference signal to be used for analysis for separation of the skin blood flow component.

The positional relation between a light irradiation means and a photo-detection means is input by the input unit 107 and the analysis unit 106 calculates a combination of the priority measurement point with the subsidiary measurement point from the input positional relation between the light irradiation means and the photo-detection means. Then, the display unit 109 displays the combination of the priority measurement point with the subsidiary measurement point in the same measurement unit in the form of a table and so forth and further displays a result of analysis by each data set by using the measurement unit consisting of one or the plurality of priority measurement point(s) and one or the plurality of the subsidiary measurement point(s) as one data set. Thereby, there are such advantageous effects that it is useful for determination of validity of the range of the measurement unit and determination of validity of the distance between the priority measurement point and the subsidiary measurement point and a structural feature of the subject 10 can be acquired from calculation of the contribution ratio of the deep part signal.

In the following, examples of various probe arrangements aiming to reduce the inter-measurement-point distance of the SD distances 15, 30 mm will be described.

In FIG. 25, (a) a probe arrangement, (b) a measurement point arrangement of the SD distance 30 mm, (c) a measurement point arrangement of the SD distance 15 mm when the SD distances 15, 30 mm are to be measured are shown (an arrangement A).

In FIG. 26, (a) a probe arrangement, (b) a measurement point arrangement of the SD distance 30 mm, (c) a measurement point arrangement of the SD distance 15 mm when the SD distances 15, 30 mm are to be measured are shown (an arrangement B).

In FIG. 27, (a) a probe arrangement, (b) a measurement point arrangement of the SD distance 30 mm, (c) a measurement point arrangement of the SD distance 15 mm when the SD distances 15, 30 mm are to be measured are shown (an arrangement C).

In FIG. 28, (a) a probe arrangement, (b) a table of the number of the measurement points of each SD distance in each measurement unit when the SD distances 7.5, 15, 30 mm are to be measured are shown.

Incidentally, in FIG. 25 to FIG. 28, the symbol 501 denotes the probe for light transmission, the symbol 502 denotes the probe for light reception, a symbol 505 denotes a probe for light transmission (attenuation: large), a symbol 506 denotes a probe for light reception (attenuation: large). In addition, the symbol 507 denotes the measurement point of the SD distance 30 mm, the symbol 508 denotes the measurement point of the SD distance 15 mm.

In FIG. 25 to FIG. 28, the maximum values of the inter-measurement-point distances of the different SD distances are respectively, 7.5 mm, 5 mm, 15mm, 11 mm. In FIG. 28, in particular, the arrangement is made such that at least one measurement point of each of SD 7.5, 15, 30 mm is included in each measurement unit (ch) and it is useful for the purpose of calculation and so forth of the brain contribution ratio. It is necessary to appropriately select the probe arrangements shown from FIG. 16 to FIG. 28, depending on the number of probes that a system has, a target living body area, the analysis method to be applied. For example, in a case of optical brain function measurement, the maximum values of the inter-measurement-point distances of the different SD distances may be made large if the influence of the brain blood flow and the skin blood flow has wide-ranging property, and it will be desirable to make the maximum values of the inter-measurement-point distances of the different SD distances smaller than the expected size of an active area if changes in brain blood flow and skin blood flow have locality.

In FIG. 29, a result of the detected light amounts at each measurement point and SD distance is shown as an example of display.
An automatic gain setting result 139 at the measurement points of the SD distance 30 mm is shown on an upper part and an automatic gain setting result 140 at the measurement points of the SD distance 15 mm is shown on a lower part. A display method is as shown in a legend 135. A display 136 that a cell at a measurement position had been displayed by being blacked out so as to indicate that the detected light amount is strong, a display 137 that a cell at a measurement position had been filled with gray and a circle (O) had been displayed therein so as to indicate that the detected light amount is appropriate, a display 138 that a cell at a measurement position had been displayed by being filled with white so as to indicate that the detected light amount is weak were used. Since the result of the detected light amounts greatly depends on the mounting state of the probe, when the detected light amounts become small at some measurement points, improvement becomes possible by using the light attenuation amount adjusting means 14. In that case, it becomes possible to again adjust the detector gain by changing the light attenuation amount and thereafter depressing a retry button 134 for gain adjustment.

Industrial Applicability

According to the present invention, in the biophotonic measurement apparatus which can be used as medical and laboratory equipment or used for confirmation of education result /rehabilitation result, home health management, market researches such as commodity monitoring and so forth, and further tissue oxygen saturation degree measurement and muscle oxygen metabolism measurement by the same method, it can be utilized for stabilizing the light reception sensitivity on each subject/each region. In particular, in the apparatus using the probe having the plurality of the irradiation - detector distances (the SD distances) for the purpose of separating the surface layer and deep layer components and so forth, it can be utilized as a means for improving the measurement accuracy.

Reference Signs List

10: subject
12: irradiation point
13: detection point
14: light attenuation amount adjusting means
20: apparatus main body
30: light
40: waveguide path
50: light transmitter
60: light receiver
101: light source
102: photodetector
103: light source drive unit
104: amplifier
105: analog-digital converter
106: control/analysis unit
107: input unit
108: memory unit
109: display unit
113: OK button
114: cancel button
134: retry button for gain adjustment
135: legend
136: display indicating that detected light amount is strong
137: display indicating that detected light amount is appropriate
138: display indicating that detected light amount is weak
139: automatic gain setting result at measurement point of SD distance 30 mm
140: automatic gain setting result at measurement point of SD distance 15 mm
201: electric signal

202: light source and detector built-in circuit
203: adjustment amount
204: housing
205: manual adjustment knob
206: semiconductor laser (Laser Diode, LD)
207: photodiode (PD)
208: color-dependent display
209: intermediate connector
210: optical fiber insertion port (attenuation amount is small)
211: optical fiber insertion port (attenuation amount is medium
212: optical fiber insertion port (attenuation amount is large)
213: insertion depth adjustment part
214: insertion port
215: absorbing or scattering medium
216: waveguide hole (attenuation amount is large)
217: waveguide hole (attenuation amount is medium)
218: waveguide hole (attenuation amount is small)
403: optical fiber
501: probe for light transmission
502: probe for light reception
503: probe holder
504: integrated type probe of probe for light transmission and probe for light reception
505: probe for light transmission (attenuation: large)
506: probe for light reception (attenuation: large)
507: measurement point of SD distance 30 mm
508: measurement point of SD distance 15 mm
509: measurement point of SD distance 5 mm
510: measurement point of SD distance 16.8 mm
601: actually measured component weight
602: X section of weighted straight line calculated from actual measurement data
603: gray matter reached minimum SD distance
604: gray matter average effective optical path length
605: non-use SD distance zone
606: primary straight line that has fitted weight at measurement point of SD distance of at least 12 mm
607: straight line having inclination of zero degrees

## Claims

1.  A biophotonic measurement apparatus, comprising:

    one or a plurality of light irradiation means for irradiating light to a subject;
    one or a plurality of photo-detection means for detecting light that has been irradiated from the light irradiations means to an irradiation point on the subject and propagated in the subject at a detection point on the subject;
    a light attenuation amount adjusting means disposed on a light propagation path between a light source element included in the light irradiation means and the subject, or between a light receiving element included in the photo-detection means and the subject;

an analysis unit for analyzing a signal obtained by the photo-detection means; and

a display unit for displaying a result of analysis by the analysis,

wherein the light irradiation means and the photo-detection means are respectively arranged on the subject such that an SD distance defined as a distance between the irradiation point and the detection point on the subject is given in two or more kinds,

the analysis unit analyzes signals by light from the light irradiation means located at positions of two or more kinds of the SD distances that at least one of the photo-detection means detects respectively and calculates a light attenuation adjustment amount for setting respective received light amounts within a predetermined range, and

the light attenuation amount adjusting means makes attenuation amounts of light from the light irradiation means located at the positions of two or more kinds of the SD distances that the photo-detection means detects respectively adjustable by changing an amount of light that is incident uopn the photo-detection means.

2. The biophotonic measurement apparatus according to claim 1, wherein the display unit displays the light attenuation adjustment amount calculated by the analysis unit.

3. The biophotonic measurement apparatus according to claim 2, wherein the display unit displays the light attenuation adjustment amount as a difference in color.

4. The biophotonic measurement apparatus according to claim 2, wherein the display unit is arranged in the same housing as the light attenuation amount adjusting means.

5. The biophotonic measurement apparatus according to claim 1, wherein the light attenuation amount adjusting means automatically adjusts a detected light amount(s) by the one or plurality of photo-detection means on the basis of the SD distance or the light attenuation adjustment amount calculated by the analysis unit.

6. The biophotonic measurement apparatus according to any one of claims 1 to 5, wherein the light attenuation amount adjusting means adjusts a connection loss when light is propagated between the light source element and the subject, or between the light receiving element and the subject.

7. The biophotonic measurement apparatus according to claim 6,
wherein a part of the light propagation path is an optical fiber,
and
the light attenuation amount adjusting means is disposed on an optical fiber joint part for optically coupling together a plurality of the optical fibers and adjusts the connection loss when light is propagated between the optical fibers.

8. The biophotonic measurement apparatus according to claim 7,
wherein the light attenuation amount adjusting means has a means for changing a positional relation between end faces of two optical fibers on the optical fiber joint part.

9. The biophotonic measurement apparatus according to claim 8, wherein the means for changing the positional relation between the end faces of the two optical fibers includes an electromagnetic control means, preferably, a piezoelectric element or a piezoelectric actuator.

10. The biophotonic measurement apparatus according to claim 7, wherein the light attenuation amount adjusting means changes the kind or the shape of a medium on a light propagation path between the end faces of two optical fibers on the optical fiber joint part.

11. The biophotonic measurement apparatus according to any one of claims 1 to 5, wherein the analysis unit extracts one or a plurality of separation component(s)from a plurality of pieces of measurement data measured by a combination of the light irradiation means with the photo- detection means by using a signal separation method.

12. The biophotonic measurement apparatus according to any one of claims 1 to 5,
wherein when a predetermined SD distance has been defined as a priority SD distance, and a region to be measured by one light irradiation means and one photo-detection means or a position representing the region has been defined as a measurement point corresponding to the light irradiation means and photo-detection means concerned,
the light irradiation means and the photo-detection means are arranged such that a measurement point (a subsidiary measurement point) corresponding to another SD distance is located at a distance of within 40 mm, more preferably, at a distance of within 20 mm from a measurement point (a priority measurement point) corresponding to the priority SD distance,

the analysis unit puts the priority measurement point and the subsidiary measurement point corresponding to the priority measurement point together and analyzes it as one data set, and

the display unit displays a result of analysis of one or a plurality of data set(s) per data set.

13. The biophotonic measurement apparatus according to claim 12, comprising:

an input means for inputting the positional relation between the light irradiation means and the photo-detection means,

wherein the analysis unit calculates a combination of the priority measurement point with the subsidiary measurement point from the positional relation between the light irradiation means and the photo-detection means, and

the display unit displays the combination in the form of a chart.

14. The biophotonic measurement apparatus according to any one of claims 1 to 5,

wherein the analysis unit estimates a head structure of the subject from a light amount detected under a condition of three or more kinds of the SD distances and a time-dependent change thereof, or a weight and an amplitude across each SD distance of each component obtained by a signal separation method.

15. A biophotonic measurement method using the biophotonic measurement apparatus according to claim 1, comprising:

the step of measuring light that has been propagated in a subject at least one measurement point of less than 10 mm, preferably not more than 8 mm in SD distance, and two or more measurement points of at least 10 mm, preferably at least 12 mm in SD distance;

the step of obtaining a primary straight line using weighted values of two or more measurement points of at least 10 mm in SD distance and obtaining the SD distance corresponding to a weight of zero in a chart that plots the SD distance and a weighted value of each component obtained by a signal separation method;

the step of obtaining a straight line that is parallel with an axis using the weighted value of the measurement point of less than 10 mm in SD distance and the step of obtaining the SD distance of an intersection point of the straight line that is parallel with the axis and the primary straight line as a gray matter reached minimum SD distance, and

the step of calculating a brain contribution ratio from the SD distance corresponding to the weight of zero and the gray matter reached minimum SD distance.

# FIG. 1

EP 2 891 457 A1

# FIG. 2

# FIG. 3

# FIG. 4

ch1 ch2 ch3 ch4

+1  −1  +2   0

# FIG. 5

# FIG. 6

# FIG. 7

# FIG. 8

# FIG. 9

# FIG. 10

# FIG. 11

# FIG. 12

```
                    ┌─────────────┐
                    │    START    │
                    └──────┬──────┘
                           │
                           ▼
         ┌──────────────────────────────────┐
         │    MOUNT PROBE ONTO SUBJECT       │ ～ S101
         └──────────────────┬───────────────┘
                            │
                            ▼
         ┌──────────────────────────────────┐
         │   IRRADIATE LASER AND MEASURE     │ ～ S102
         │      DETECTED LIGHT AMOUNT        │
         └──────────────────┬───────────────┘
                            │
                            ▼                          S103
              ┌──────────────────────────────┐
              │   IS DETECTED LIGHT AMOUNT    │
              │ WITHIN PREDETERMINED RANGE?   │   NO
              └──────────────────────────────┘
                      YES                                     S105
                                                ┌──────────────────────┐
                                    YES         │      AT LEAST         │
                                   ◄────────────│   THRESHOLD VALUE?    │
                                                └──────────────────────┘
                                                          NO

   ┌──────────────────┐   ┌──────────────────┐   ┌──────────────────┐
   │ DISPLAY "NORMAL" │   │ DISPLAY "LARGE"  │   │ DISPLAY "SMALL"  │
   └──────────────────┘   └──────────────────┘   └──────────────────┘
          ～ S104               ～ S106                ～ S107

                           ┌─────────────┐
                           │     END     │
                           └─────────────┘
```

# FIG. 13

## (A)

## (B)

# FIG. 14

# FIG. 15

```
                    ┌─────────────┐
                    │    START    │
                    └─────────────┘
                           │
                           ▼
    ┌───────────────────────────────────────────┐
    │    OBTAIN STRAIGHT LINE (L1) USING WEIGHTED│──── S201
    │       VALUE OF AT LEAST ABOUT SD 12 mm     │
    │         AND DENOTE x SECTION AS Xs         │
    └───────────────────────────────────────────┘
                           │
                           ▼
    ┌───────────────────────────────────────────┐
    │      OBTAIN STRAIGHT LINE (L2) PARALLEL    │──── S202
    │     WITH X-AXIS USING WEIGHTED VALUE       │
    │      OF NOT MORE THAN ABOUT SD 8 mm        │
    └───────────────────────────────────────────┘
                           │
                           ▼
    ┌───────────────────────────────────────────┐
    │    DENOTE x-COORDINATE OF INTERSECTION     │──── S203
    │       POINT OF L1 WITH L2 AS Xsgray        │
    └───────────────────────────────────────────┘
                           │
                           ▼
    ┌───────────────────────────────────────────┐
    │    CALCULATE BRAIN CONTRIBUTION RATIO      │──── S204
    │   OF PART CONCERNED FROM Xs AND Xsgray     │
    └───────────────────────────────────────────┘
                           │
                           ▼
                    ┌─────────────┐
                    │     END     │
                    └─────────────┘
```

# FIG. 16

(A)

501 — Source
502 — detector
504 — Source+detector (SD=5mm)

30mm

(B)

509 — MEASUREMENT POINT OF SD DISTANCE 5 mm
508 — MEASUREMENT POINT OF SD DISTANCE 15 mm
507 — MEASUREMENT POINT OF SD DISTANCE 30 mm

# FIG. 17

(A)

501 — ⬤ Source

502 — ◯ detector

(B)

508 — ◦ MEASUREMENT POINT OF SD DISTANCE 15 mm
507 — ▫ MEASUREMENT POINT OF SD DISTANCE 30 mm

# FIG. 18

## (A)

```
        501 ──○  Source

        502 ──○  detector

        ──  PAIR OF SD DISTANCES 15 mm
```

## (B)

```
    508 ──○  MEASUREMENT POINT OF SD DISTANCE 15 mm
    507 ──□  MEASUREMENT POINT OF SD DISTANCE 30 mm
```

# FIG. 19

## (A)

501 ⬤ Source

502 ◯ detector

## (B)

508 ◯ MEASUREMENT POINT OF SD DISTANCE 15 mm

507 ☐ MEASUREMENT POINT OF SD DISTANCE 30 mm

# FIG. 20

## (A)

Source
detector

## (B)

510 ━━●  MEASUREMENT POINT OF SD DISTANCE 16.8 mm
508 ━━○  MEASUREMENT POINT OF SD DISTANCE 15 mm
507 ━━□  MEASUREMENT POINT OF SD DISTANCE 30 mm

# FIG. 21

## (A)

127mm

42.4mm

501 — ● Source

502 — ○ detector

—— PAIR OF SD DISTANCES 30 mm

## (B)

|   1  |   2  |   3  |   4  |   5  |   6  |
|------|------|------|------|------|------|
|   7  |   8  |   9  |  10  |  11  |  12  |

508 — ○ MEASUREMENT POINT OF SD DISTANCE 15 mm

507 — □ MEASUREMENT POINT OF SD DISTANCE 30 mm

# FIG. 22

## (A)

170mm

42.4mm

501 — ⬤ Source

502 — ◯ detector

— PAIR OF SD DISTANCES 30 mm

## (B)

| 1 | 2 | 3 | 4 | 5 | 6 | 7 | 8 |
| 9 | 10 | 11 | 12 | 13 | 14 | 15 | 16 |

508 — ◯ MEASUREMENT POINT OF SD DISTANCE 15 mm

507 — ☐ MEASUREMENT POINT OF SD DISTANCE 30 mm

# FIG. 23

## (A)

504 — ◐ LIGHT SOURCE & DETECTOR OF SD DISTANCE 5 mm
501 — ☐ LIGHT SOURCE
502 — ⬟●■⬢ DETECTOR

## (B)

509 — ● MEASUREMENT POINT OF SD DISTANCE 5 mm
508 — ○ MEASUREMENT POINT OF SD DISTANCE 15 mm
507 — ☐ MEASUREMENT POINT OF SD DISTANCE 30 mm

# FIG. 24

## (A)

## (B)

# FIG. 25

(A)

(B)

(C)

# FIG. 26

(A)

(B)

(C)

# FIG. 27

## (A)

24  16  18

22  16

15        18

17  26

15  17  28

501

505

502

## (B)

507

16  20

19  23

## (C)

7  21

508

18  21

# FIG. 28

(A)

(B)

| S–D (mm) | 7.5 | 15 | 30 |
|----------|-----|----|----|
| ch1 | 1 | 1 | 1 |
| ch2 | 1 | 1 | 1 |
| ch3 | 3 | 1 | 1 |
| ch4 | 3 | 1 | 1 |
| ch5 | 3 | 1 | 1 |
| ch6 | 3 | 1 | 1 |
| ch7 | 1 | 1 | 1 |
| ch8 | 1 | 1 | 1 |
| SUM TOTAL (sum) | 16 | 8 | 8 |

# FIG. 29

## INTERNATIONAL SEARCH REPORT

| International application No. |
| --- |
| PCT/JP2013/068788 |

**A.   CLASSIFICATION OF SUBJECT MATTER**
*A61B10/00*(2006.01)i, *A61B5/1455*(2006.01)i

According to International Patent Classification (IPC) or to both national classification and IPC

**B.   FIELDS SEARCHED**

Minimum documentation searched (classification system followed by classification symbols)
A61B10/00, A61B5/1455

Documentation searched other than minimum documentation to the extent that such documents are included in the fields searched
Jitsuyo Shinan Koho         1922-1996   Jitsuyo Shinan Toroku Koho   1996-2013
Kokai Jitsuyo Shinan Koho   1971-2013   Toroku Jitsuyo Shinan Koho   1994-2013

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
| --- | --- | --- |
| Y | WO 2012/005303 A1  (Hitachi Medical Corp.), 12 January 2012 (12.01.2012), entire text; all drawings & US 2013/0102907 A1     & EP 2591732 A & CN 103153198 A | 1-15 |
| Y | JP 2003-207443 A  (Shimadzu Corp.), 25 July 2003 (25.07.2003), claims 1 to 4; paragraphs [0056] to [0060]; fig. 5 to 7 (Family: none) | 1-15 |
| Y | JP 2004-333568 A  (Fujikura Ltd.), 25 November 2004 (25.11.2004), paragraph [0002] (Family: none) | 1-15 |

☒  Further documents are listed in the continuation of Box C.          ☐  See patent family annex.

| | |
| --- | --- |
| *     Special categories of cited documents:<br>"A"   document defining the general state of the art which is not considered    to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    25 July, 2013 (25.07.13) | Date of mailing of the international search report<br>    06 August, 2013 (06.08.13) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2013/068788 |

C (Continuation).　DOCUMENTS CONSIDERED TO BE RELEVANT

| Category* | Citation of document, with indication, where appropriate, of the relevant passages | Relevant to claim No. |
| --- | --- | --- |
| Y | JP 2004-102172 A　(Sumitomo Electric Industries, Ltd.), 02 April 2004 (02.04.2004), entire text; all drawings (Family: none) | 1-15 |
| Y | JP 2004-309791 A　(Sumitomo Electric Industries, Ltd.), 04 November 2004 (04.11.2004), entire text; all drawings (Family: none) | 1-15 |

Form PCT/ISA/210 (continuation of second sheet) (July 2009)

## REFERENCES CITED IN THE DESCRIPTION

**Patent documents cited in the description**

- JP 2010240298 A
- JP 2006200943 A
- WO 2012005303 A
- WO 2010150751 A

**Non-patent literature cited in the description**

- **A. MAKI et al.** Spatial and temporal analysis of human mot or activity using noninvasive NIR topography. *Medical Physics,* 1995, vol. 22 (12), 1997-2005
- **T. FUNANE et al.** Discrimination of Skin Blood Flow Using Multi-Distance Probe and Independent Component Analysis in Optical Brain Function Monitoring. *The papers of Technical Meeting on Optical and Quantum Devices, IEE, Japan,* 2011, 17-22
- **I. ODA et al.** Near Infrared Imager with a Flexible Source-Detector Arrangement and a New Detection Gain Control. *Optical Review,* 2003, vol. 10 (5), 422-426